(19) Europäisches Patentamt / European Patent Office / Office européen des brevets

(11) **EP 4 035 175 B1**

(12) **EUROPEAN PATENT SPECIFICATION**

(45) Date of publication and mention
of the grant of the patent:
**02.07.2025 Bulletin 2025/27**

(21) Application number: **20772331.3**

(22) Date of filing: **23.09.2020**

(51) International Patent Classification (IPC):
**G16H 50/20** (2018.01)   **G16H 20/60** (2018.01)
**G16H 50/70** (2018.01)   **G16B 20/00** (2019.01)
**G16B 40/20** (2019.01)

(52) Cooperative Patent Classification (CPC):
**G16H 50/20; G16B 20/00; G16B 40/20;
G16H 20/60; G16H 50/70**

(86) International application number:
**PCT/EP2020/076497**

(87) International publication number:
**WO 2021/058523 (01.04.2021 Gazette 2021/13)**

(54) **PREDICTING THE RESPONSE OF A MICROBIOTA TO DIETARY FIBRES**

VORHERSAGE DER REAKTION EINER MIKROBIOTA AUF DIÄTFASERN

PRÉDICTION DE LA RÉPONSE D'UN MICROBIOTE AUX FIBRES ALIMENTAIRES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **23.09.2019 EP 19198865**

(43) Date of publication of application:
**03.08.2022 Bulletin 2022/31**

(73) Proprietor: **MYOTA GMBH
10119 Berlin (DE)**

(72) Inventor: **GURRY, Thomas Jérôme
1223 Cologny (CH)**

(74) Representative: **KATZAROV S.A.
Geneva Business Center
12 Avenue des Morgines
1213 Petit-Lancy (CH)**

(56) References cited:
**WO-A1-2019/046372     US-A1- 2018 357 375**

• TINGTING CHEN ET AL: "Fiber-utilizing capacity varies in Prevotella- versus Bacteroides-dominated gut microbiota", SCIENTIFIC REPORTS, vol. 7, no. 1, 1 June 2017 (2017-06-01), XP055676533, DOI: 10.1038/s41598-017-02995-4
• RILEY L HUGHES ET AL: "The Role of the Gut Microbiome in Predicting Response to Diegt and the Development of Precision Nutrition Models", ADVANCES IN NUTRITION, vol. 10, no. 6, 21 June 2019 (2019-06-21), United States, pages 953 - 978, XP055676903, ISSN: 2161-8313, DOI: 10.1093/advances/nmz022

**Description**

**FIELD OF THE INVENTION**

The invention relates to the human gut microbiota and its metabolic capabilities. In particular to a method evidencing the functional heterogeneity in the fermentation capabilities of the healthy human gut microbiota. More particularly, the invention provides a method for predicting a response to different dietary fibres based on the analysis and measuring of the fermentation or metabolic capabilities of a subject's gut microbiota as well as a computer software product and an apparatus for predicting a response of a subject to different dietary fibres.

**BACKGROUND OF THE INVENTION**

The symbiotic relationship between host and gut microbiota is intimately related to host diet. For example, the majority of the caloric intake of ruminants is derived from the microbial fermentation of otherwise indigestible polysaccharides in their diet. In humans, fermentation of dietary fibers and other Microbiota Accessible Carbohydrates (MACs) only accounts for a fraction of total caloric intake, but the resulting metabolites also play other important physiological roles (Davie, 2003; Kuo, 2013). In particular, the Short Chain Fatty Acids (SCFAs) acetate, propionate and butyrate (the major by-products of the microbial fermentation of dietary fibers in the gut) exert a number of forces on the host's physiology. While it has long been known that butyrate serves as the dominant energy source for colonocytes (Roediger, 1980), and is therefore critically important to maintaining a healthy gut, it has been shown more recently that increases in microbial acetate production and turnover have been shown to activate glucose-stimulated insulin secretion via a gut-brain axis mediated process, which can lead to insulin resistance and subsequent obesity, in addition to increasing a patient's risk of developing Type 2 diabetes (Perry et al., 2016). Propionate and butyrate, in contrast, activate gluconeogenesis, which has beneficial effects to host metabolism and plasma glucose regulation (De Vadder et al., 2014; Zhao et al., 2018). Indeed, increased production of butyrate has been causally related to improved insulin response after an oral glucose-tolerance test, whereas abnormalities in propionate production were linked to increased risk of Type-2 Diabetes (Sanna et al., 2019).

While many of these effects are mediated by short chain fatty acid receptors in the gut epithelium, a critical property of colonic SCFAs is their activity as histone deacetylase (HDAC) inhibitors (Davie, 2003). This ability to regulate gene expression in host cells has associated microbially-derived SCFAs with a growing list of clinical indications. Butyrate, in particular, has been proposed to exert anti-inflammatory pressure on the host's immune system through several mechanisms, including differentiation of regulatory and IL-10-producing T cells, downregulation of IL-6 production, pro-inflammatory T cell apoptosis, and suppression of IFN-γ-mediated inflammation in the colonic epithelium (Zimmerman et al., 2012). These data complement associations found between depletions in butyrate-producing organisms such as *Faecalibacterium prausnitzii* and Inflammatory Bowel Disease (IBD) (Machiels et al., 2013) to indicate that SCFAs likely play important roles in the disease etiology. Moreover, the fact that SCFAs produced by the microbiota in the colon can be absorbed into the blood stream suggests that their effects on gene expression may transcend the gut and affect distal tissues in ways that currently poorly understood. It is therefore of significant clinical interest to improve our quantitative understanding of SCFA production in the gut, in order that it may be modulated towards a particular clinical outcome.

SCFAs are the chemical end-result of the fermentation of polysaccharides through a variety of biochemical pathways in the gut metagenome. These begin with the hydrolysis of these complex dietary polysaccharides to their constituent monosaccharides by members of the microbiota encoding the appropriate Polysaccharide Utilization Loci (PULs) and Glycoside Hydrolases (GHs), which differ widely between different bacterial species and even strains within a species (Sonnenburg et al., 2010). These monosaccharides can then be fermented by a number of fermentation pathways to ultimately result in acetate, propionate or butyrate. Importantly, there is cross-talk between fermentation pathways: for example, the most prevalent butyrate-producing pathway in the human gut involves the enzyme butyryl-CoA : acetate-CoA transferase (Duncan et al., 2002), which exchanges an butyrate moiety for an acetate moiety and releases free butyrate. Thus, the pool of available acetate affects the production of both propionate and butyrate. Other products of bacterial fermentation can also serve as intermediates in SCFA production: lactate, in particular, can act as a substrate for further fermentation into acetate, propionate and butyrate. Moreover, the SCFA producing organisms may not be able to directly ferment a specific polysaccharide themselves; instead, they rely on the preliminary degradation of these dietary fibers into hexoses and pentoses by other bacteria encoding these carbohydrate active enzymes. This gives rise to a host of cooperative microbial networks that act in concert to produce the overall SCFA profile present in an individual's colon. Thus, the combination of dietary inputs (which dietary fibers, in which quantities) and the composition of an individual's microbiota together dictate what ratios and absolute quantities of SCFAs are produced in their gut, with potentially important effects on physiology. Since SCFAs have been implicated in a variety of different physiological and disease-related mechanisms, it is unique and promising target.

There exists many disclosures extremely broad in nature, referring either to features diagnostic of a specific disease indication from stool microbiota sequencing, or to probiotic formulations or bacterial consortia for treating specific conditions. For example, specific glycan polymers have been disclosed for the treatment of disease. In WO2018106845 A1 (KALEIDO BIOSCIENCES INC [US]) it is disclosed compositions of glycan polymers and methods of making and manufacturing the same. Also provided are methods of treating a disease or disorder with a glycan polymer preparation.

Existing stool sampling kits for the purpose of sequencing the microbiota do not provide functional predictions of experimentally measurable and validated quantities that pertain to human health, with the exception of WO 2015/166489 A2 (YEDA RES & DEV [IL]) which provides a method of predicting a response of a subject to food in particular through glycemic response. The method comprises: selecting a food to which a response of the subject is unknown; accessing a first database having data describing the subject but not a response of the subject to the selected food; accessing a second database having data pertaining to responses of other subjects to foods, the responses of the other subjects including responses of at least one other subject to the selected food or a food similar to said selected food; and analyzing the databases based on the selected food to estimate the response of the subject to the selected food. However, this document focuses exclusively on post-prandial glucose response (PPGR), and uses machine learning to predict PPGR to different foods from metagenomics DNA sequencing. Moreover, this technology requires a blood sample, which cannot be sent.

WO 2019/046372 AI (UNIV MICHIGAN REGENTS [US]) discloses compositions and methods for increasing butyrate production in a subject. In particular, this document provides compositions, probiotic compositions, and combinations thereof that promote butyrate production in a subject. Also disclosed is a method of increasing butyrate levels in the intestine of a subject, comprising: administering a carbohydrate source and at least one first bacteria selected from the group consisting of bacteria belonging to the taxons identified as *Bifidobacterium* spp. *, Clostridium* seq 176, sequence 100, and *Ruminococcus bromii.* This document describes formulations that boost butyrate production in a subject, regardless of their microbiota composition. In particular, it discloses administering a carbohydrate source and a bacterium together to reach the desired effect.

TINGTING CHEN ET AL: "Fiber-utilizing capacity varies in Prevotella-versus Bacteroides-dominated gut microbiota", SCIENTIFIC REPORTS, vol. 7, no. 1, 1 June 2017 (2017-06-01) discloses that the gut microbiota of individuals are dominated by different fiber-utilizing bacteria, which ferment dietary fiber into short chain fatty acids (SCFAs) known to be important for human health. It was shown that the dominance of Prevotella versus Bacteroides in fecal innocula, identified into two different enterotypes, differentially impacts in vitro fermentation profiles of SCFAs from fibers with different chemical structures. In a microbiome of the Prevotella enterotype, fructooligosaccharides, and sorghum and corn arabinoxylans significantly promoted one single Prevotella OTU with equally high production of total SCFAs with propionate as the major product. Conversely, in the Bacteroides-dominated microbiota, the three fibers enriched different OTUs leading to different levels and ratios of SCFAs. This document shows how individual differences in two enterotypes cause distinctly different responses to dietary fiber. Micro biota dominated by different fiber-utilizing bacteria may impact host health by way of producing different amounts and profiles of SCFAs from the same carbohydrate substrates. This document focuses on a commonly used dichotomy in the field, namely Prevotella- versus Bacteroides-dominated microbiota compositions, and reports analyses according to these so-called 'enterotypes', where one donor is chosen from each type for the fermentation experiments. While the authors' data indicate differences in fermentation profiles from the two donors, these data do not per se demonstrate that microbiota composition can predict SCFA production from specific fibers. Instead, they compare responder OTUs and SCFA production between the two donor microbiotas when challenged with the same fibers, and ascribe these differences to one microbiota being dominated by Prevotella, and the other dominated by Bacteroides. One cannot from these data concur that knowledge of a microbiota composition can predict the metabolic response to specific fibers, since there was only N=1 example in each of the two categories and there are no analyses, statistical or otherwise, indicating that a model taking microbiota composition of new samples as input can return predictions about SCFA production in those samples were they to be challenged with different fibers.

US 2018/357375 A1 (CUTCLIFFE COLLEEN [US] ET AL) provides methods of determining metabolic maps and identifying presence of and estimating abundances of microbiome metabolic pathways in an individual toward customized microbial therapy. In an aspect, the present disclosure provides a method of determining an abundance of a metabolic pathway from a sample comprising a population of a plurality of different organisms.

This method can be used to estimate the value of a given metabolic pathway in a microbiota, including an SCFA production pathway. These data can be used to predict the potential of the microbiota's SCFA production capacity, in that the higher the abundance of the pathway, the higher the maximum capable metabolic flux through that pathway. However, there is no reference to estimating the production rate of a given metabolite from a given input, and there is no consideration towards the fact that a specific pathway of abundance computed using the aforementioned method can produce different metabolic fluxes in response to different inputs (dietary or otherwise) as a function of upstream processes, which in the case of SCFA production depends on the presence of upstream glycoside hydrolases or polysaccharide utilization loci capable of degrading the specific fiber in question into a chemical entity that acts as an input into the specific SCFA production pathway.

RILEY L HUGHES ET AL: "The Role of the Gut Microbiome in Predicting Response to Diet and the Development of Precision Nutrition Models", ADVANCES IN NUTRITION, vol. 10, no. 6, 21 June 2019 (2019-06-21), pages 953-978 reports that health care is increasingly focused on health at the individual level. In the rapidly evolving field of precision nutrition, researchers aim to identify how genetics, epigenetics, and the microbiome interact to shape an individual's response to diet. With this understanding, personalized responses can be predicted and dietary advice can be tailored to the individual. With the integration of these complex sources of data, an important aspect of precision nutrition research is the methodology used for studying interindividual variability in response to diet. This article investigates the contribution of the gut microbiota to interindividual variability in response to diet. The methods used by researchers to design and carry out such studies as well as the statistical and bioinformatic methods used to analyze results are reviewed. This article also reviews the findings of these studies, discusses gaps in the current knowledge, and summarizes directions for future research. Taken together, this review summarizes the current state of knowledge and provide a foundation for future research on the role of the gut microbiome in precision nutrition. This document references several articles that describe the Prevotella to Bacteroides (P:B) ratio as associated with response to fiber interventions, as well as the abundance of specific bacterial clades (e.g. *Bifidobacteria).* However, this 'response to fiber' refers to responses to fibers as total SCFA outputs in response to consumption of dietary fiber (i.e. the overall production of SCFAs in response to fiber), and does not refer to specific associations between response to individual fibers and individual metabolites/SCFAs. There is no link between the production rates of specific SCFA/metabolites in response to specific fiber structures, and the relative abundance of specific metagenomic sequences derived from a combination of genes and metabolic pathways.

There are still no diagnostic available solution for measuring or predicting Short Chain Fatty Acid (SCFA) production (as well as intermediate fermentation metabolites such as oligosaccharides, monosaccharides, lactate, formate and succinate) in response to different dietary compounds.

It is an object of the present invention to better understand the extent of differential fermentation capabilities in the microbiota of the healthy human population, and present an *ex vivo* framework for measuring the production of SCFAs of an individual's stool microbiota in response to challenge with specific dietary fibers. Performing these experiments *ex vivo* allows Applicants to quantify SCFA accumulation accurately and circumvents the technical difficulties associated with measurements of SCFA production in vivo (invasiveness) or SCFA measurements from raw stool upon passage (unknown extent of SCFA absorption by the gut epithelium).

## BRIEF DESCRIPTION OF THE INVENTION

The human gut microbiota is known for its highly heterogeneous composition across different individuals. However, relatively little is known about functional differences in its ability to ferment polysaccharides into Short Chain Fatty Acids (SCFAs). Through *ex vivo* measurements from healthy human donors, Applicants show that individuals vary markedly in their microbial metabolic phenotypes (MMPs), mirroring differences in their microbiota composition, and resulting in the production of different quantities and proportions of SCFAs from the same inputs. Applicants also show that aspects of these MMPs can be predicted from composition using 16S rRNA sequencing. From experiments performed using the same dietary fibers *in vivo,* Applicants demonstrate that an ingested bolus of fiber is almost entirely consumed by the microbiota upon passage. Applicants leverage these *ex vivo* data to construct a model of SCFA production and absorption *in vivo,* and argue that interindividual differences in quantities of absorbed SCFA are directly related to differences in production. Taken together, these data evidence that personalized dietary fiber supplementation based on an individual's MMP is an attractive and cost effective therapeutic strategy for treating diseases associated with SCFA production.

The technical problem to be solved comes in several layers. The first layer is the ability to predict the metabolic capabilities of an individual's microbiota from nucleic acids or metabolites present in a stool sample, opening up the possibility of a mailer diagnostic kit that can maintain the sample 'stable' for sufficiently long that it can be shipped from anywhere in the world to a sequencing or treatment facility. This is achieved for example by training machine learning algorithms on a database of experimental measurements obtained from live stool microbiotas. The second layer is to formulate personalized dietary recommendations from these predictions, for example which specific dietary fibres should be consumed to maximize the production of a given Short Chain Fatty Acid. The third layer is to apply these personalized dietary recommendations to a specific clinical indication, e.g. maximizing butyrate production for example in Inflammatory Bowel Disease or Alzheimer's disease patients.

One of the objects of the present invention is to provide a method for predicting a response to different dietary fibres from nucleic acid sequences, based on the analysis of the fermentation or metabolic capabilities of a subject's gut microbiota assessed by its short chain fatty acid (SCFA) production rate in response to said different dietary fibres, the method being applied to abundance data of specific microbiota-derived nucleic acids obtained from prior collected stabilized biological sample of said subject, the method comprising the steps of:

- isolating and extracting said specific microbiota-derived nucleic acids as well as microbiota-derived metabolites from said stabilized biological sample, wherein the specific sequences of said specific microbiota-derived nucleic acids are identified from a training database containing metagenomics sequencing data from healthy subjects, or subjects suffering from a particular disease, paired with *in vitro* measured data pertaining to the production rates of SCFAs and other fermentation metabolites from different dietary fibers, selected from the list comprising propionate, butyrate, acetate, lactate, formate, succinate, iso-butyrate, valerate and iso-valerate;
- determining and quantifying the relative abundance of said specific microbiota-derived nucleic acids from said stabilized biological sample to generate a first input, wherein said specific microbiota-derived nucleic acids consist in gene sequences specific to glycoside and/or polysaccharide hydrolases capable of hydrolyzing or cleaving complex polysaccharides of said different dietary fibres, as well as gene sequences specific to key enzymes from bacterial acetate, propionate and butyrate production pathways;
- applying a machine learning algorithm to predict from these specific microbiota-derived nucleic acid sequences the production rate of SCFAs and other fermentation metabolites in response to challenge with different dietary fibers so as to obtain a response to said different dietary fibres.

Another object of the present invention is to provide a computer software product, comprising a computer-readable medium in which program instructions are stored, which instructions, when read by a data processor, cause the data processor to receive data collected from a subject's sample, and to execute the method according to the invention.

A further object of the invention is to provide an apparatus for predicting a response of a subject to different dietary fibres, the apparatus comprising: a user interface configured to receive a subject's sample; a module for extracting microbiota-derived nucleic acids from said subject's sample and for quantifying and/or sequencing said nucleic acids; and a data processor having a computer-readable medium storing the computer software product of the invention.

Other objects and advantages of the invention will become apparent to those skilled in the art from a review of the ensuing detailed description, which proceeds with reference to the following illustrative drawings, and the attendant claims.

**BRIEF DESCRIPTION OF THE FIGURES**

**Figure 1:** Schematic of assay setup and sampling frequency.

**Figure 2:** 24h time traces of butyrate concentration over time in response to inulin (I), pectin (P), cellulose (Ce) and control (Ct), in two different participants. Participant C only produces butyrate from inulin, while participant G produces it from pectin as well. Both participants have linear production regimes in the 0-4h window used to calculate production rate.

**Figure 3:** Acetate, propionate and butyrate production rates from inulin (INUL) and pectin (PECT) in different participants, presented as Z-scores computed across all participants. Each row represents measurements from a single sample. SCFA production rates were measured ex vivo in mM/h for each participant in response to each condition. Production rates were computed between timepoints 2h and 4h for each condition and production rates from the control condition (no spike-in) were subtracted. Cellulose timepoints were indistinguishable from control and therefore were not presented.

**Figure 4:** AUC values for different RFCs trained either to predict high or low SCFA content in stool at baseline, or high or low SCFA production rate ex vivo in response to specific dietary fibers. High and low production is defined according to the z-score across all participants in the study.

**Figure 5:** (a) Relationship between propionate and butyrate production rates, and the relative abundance of an unassigned OTU of the Lachnospiraceae family, showing a relationship between its relative abundance and butyrate production in response to inulin, specifically. (Inul: inulin; Pect: pectin). **(b)** Similar relationship but specific to the relative abundance of a Prevotella copri OTU and propionate production in response to inulin.

**Figure 6:** shows that general features of an individual's MMP are stable over time. **(a)** Continuous production rates of each SCFA in response to inulin and pectin for two timepoints separated by at least six months, expressed as z-scores relative to the population in the dataset. **(b)** The same data, but collapsed to high or low producers of a given SCFA in response to a given fiber (dark = high, light = low).

**Figure 7:** Schematic of model parameters.

**Figure 8:** Concentration of inulin in participant stool from a previous study where participants were fed 10g of inulin on days 4, 5 and 6 against a constant (fiber-impoverished) dietary background.

**Figure 9:** Predicted quantities of butyrate absorbed versus excreted in the stool in participant H as a function of the colonic epithelial absorption rate constant, assuming a transit time of 12 hours. Values of the rate constant measured by the dialysis bag and CaCo monolayer approaches discussed in the text are shown explicitly.

**Figure 10:** Predicted amount of each SCFA absorbed (in mmol) using a transit time of 12 hours for each subject and the dialysis bag rate constant parameters.

**Figure 11:** Concentrations of inulin measured at t = 0, 2h, 4h, 6h and 24h in six separate donors (donor IDs K, S, Z, EE, 1 and 2), determined using an inulin-specific ELISA assay.

**Figure 12:** Shannon diversity index of 16S rRNA communities in each condition and each timepoint (0h, 4h, 24h).

**Figure 13:** shows the pH of the slurry over time, measured across all participants in the study (Ctrl: control, Inul: inulin, Pect: pectin, Cell: cellulose)

**Figure 14:** shows pairwise linear regressions between Bristol score (BSS: Bristol stool scale) of the sample, total SCFAs produced, and qPCR amplification cycle (CT values).

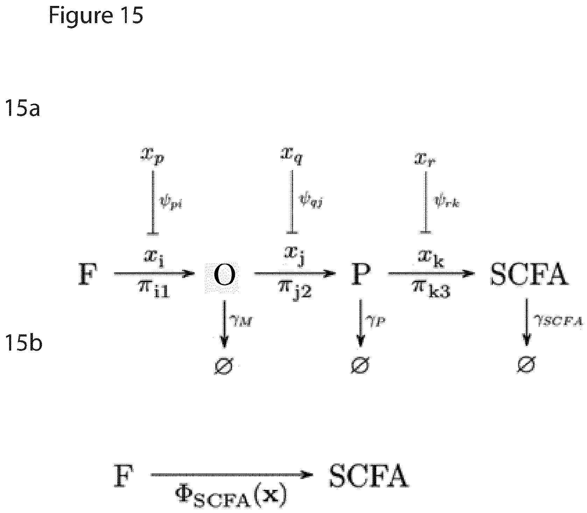

**Figure 15:** illustrates that the Microbial SCFA production proceeds through the cooperation of different members of the microbiota. (a) Schematic illustrating the generic steps involved in dietary fiber degradation. A bacterial OTU i of relative abundance $x_i$ hydrolyses the polysaccharide (dietary fiber) F into oligosaccharides O. These are then fermented into a reduced intermediate P by OTU j with relative abundance $x_j$. Finally, P may be further fermented to an SCFA by OTU k with relative abundance $x_k$. In addition, the ability of an OTU to carry out a given reaction can itself be inhibited by a separate OTU (e.g. $x_i$ is inhibited by $x_o$). **(b)** Bulk measurement of the overall production rate of a given SCFA, $\phi SCFA$ (**x**), which is itself a function of the composition of the stool microbiota, x, and corresponds to the quantities measured using Applicants' ex vivo experiments.

## DETAILED DESCRIPTION OF THE INVENTION

Although methods and materials similar or equivalent to those described herein can be used in the practice or testing of the present invention, suitable methods and materials are described below. The publications and applications discussed herein are provided solely for their disclosure prior to the filing date of the present application. Nothing herein is to be construed as an admission that the present invention is not entitled to antedate such publication by virtue of prior invention. In addition, the materials, methods, and examples are illustrative only and are not intended to be limiting.

In the case of conflict, the present specification, including definitions, will control.

Unless defined otherwise, all technical and scientific terms used herein have the same meaning as is commonly understood by one of skill in art to which the subject matter herein belongs. As used herein, the following definitions are supplied in order to facilitate the understanding of the present invention.

The term "comprise" is generally used in the sense of include, that is to say permitting the presence of one or more features or components.

As used in the specification and claims, the singular forms "a", "an" and "the" include plural references unless the context clearly dictates otherwise.

As used herein the terms "subject" or "patient" are well-recognized in the art, and, are used interchangeably herein to refer to a mammal, and, most preferably, a human. In some embodiments, the subject can be a normal subject. The term does not denote a particular age or sex. Thus, adult and newborn subjects, whether male or female, are intended to be covered. The term "dietary fibre" is well-recognized in the art, and is used interchangeably with "complex polysaccharides" or "complex carbohydrates" to refer to carbohydrates. Complex polysaccharides or other dietary fibre ingredients include inulins, fructo-oligosaccharide, resistant starch, lignin, tannin, cellulose, hemicelluloses (including xyloglucans, glucuronoxylans, arabinoglucuronoxylans, arabinoxylans, heteroxylans, mannans, and beta-glucans), psyllium, polydextrose, chitin, chitosan, pectins (including homogalacturonans/polygalacturonans, and rhamnogalacturonans I and II), arabinan, and konnyaku / konjac (glucomannan).

The term "Short Chain Fatty Acid" is well-recognized in the art, and is used interchangeably herein to refer to formate, acetate, propionate, butyrate, valerate, iso-butyrate and iso-valerate.

The term "intermediate fermentation metabolites" refer to intermediate biochemical entities in the process of fermenting complex polysaccharides and amino acids into Short Chain Fatty Acids. These include, but are not limited to oligosaccharides, monosacchrides (e.g. fructose, glucose, galacturonic acid), lactate, formate and succinate.

The term "metagenomic sequencing" refers both to shotgun sequencing of DNA isolated from a complex ecosystem (e.g. stool), and to DNA sequencing of specific marker genes (e.g. 16S rRNA sequencing) from an ecosystem.

The term "metatranscriptomic sequencing" refers to sequencing of RNA transcripts from a complex ecosystem.

The term "metabolomics" refers to measurement of one or more analytes from a sample using analytical chemistry techniques (e.g. mass spectrometry, which may or may not be coupled to a chromatographic or purification technique).

In general, the terms "prevent," "control" and "treat" encompass the prevention of the development of a disease or a symptom, including but not limited to a subject who may have a predisposition of the disease or the symptom but has yet been diagnosed to have the disease or the symptom; the inhibition of the symptoms of a disease, namely, inhibition or retardation of the progression thereof; and the alleviation of the symptoms of a disease, namely, regression of the disease or the symptoms, or inversion of the progression of the symptoms.

All types of obesity may be controlled or treated in accordance with some embodiments of the invention, including, without limitation, endogenous obesity, exogenous obesity, hyperinsulinar obesity, hyperplastic-hypertrophic obesity, hypertrophic obesity, hypothyroid obesity and morbid obesity. For example, the present embodiments can be used to slow down, stop or reverse body weight gain, specifically body fat gain, resulting in a maintenance or decrease in body weight. A decrease in weight or body fat may protect against cardiovascular disease by lowering blood pressure, total cholesterol, LDL cholesterol and triglycerides, and may alleviate symptoms associated with chronic conditions such as hypertension, coronary heart disease, type 2 diabetes, hyperlipidemia, osteoarthritis, sleep apnea and degenerative joint disease.

Metabolic syndrome, or Syndrome X, is a complex multi-factorial condition accompanied by an assortment of abnormalities including hypertension, hypertriglyceridemia, hyperglycemia, low levels of HDL-C, and abdominal obesity. Individuals with these characteristics typically manifest a prothrombotic and proinflammatory state. Available data suggest that metabolic syndrome is truly a syndrome (a grouping of risk factors).

Each of the disorders associated with metabolic syndrome are risk factors in their own right, and can promote atherosclerosis, cardiovascular disease, stroke, systemic micro and macro vascular complications and other adverse health consequences. However, when present together, these factors are predictive of increased risk of cardiovascular disease, stroke and systemic micro and macro vascular complications.

In the context of the present invention, "disorder and/or pathological condition with metabolic and/or immunological and/or inflammatory involvement" means an alteration in human energy metabolism and/or in the correct functioning of the immune system and/or in the inflammatory state (local, at the level of the intestine, and/or systemic), which results in a clinical manifestation. Said clinical manifestation is preferably: obesity, type 2 diabetes, metabolic syndrome, non-alcoholic hepatic steatosis, insulin resistance, hypercholesterolemia, deregulation of glucose metabolism, cardiovascular diseases, hypertension, Crohn's disease, ulcerative colitis, rheumatoid arthritis, diverticular diseases, irritable bowel syndrome, allergies, food intolerances, diarrhoea, constipation, colitis and enteritis.

As used herein, the term "microbiome" refers to the totality of microbes (bacteria, fungae, protists, and viruses), their genetic elements (genomes) in a defined environment. In other words "Microbiome" refers to the genetic content of the communities of microbes ("microbiota") that live in and on a subject (e.g., a human subject), both sustainably and transiently, including eukaryotes, archaea, bacteria, and viruses (including bacterial viruses (e.g., phage)), wherein "genetic content" includes genomic DNA, RNA such as ribosomal RNA and messenger RNA, the epigenome, plasmids, and all other types of genetic information. In some embodiments, microbiome specifically refers to genetic content of the communities of microorganisms in a niche.

"Microbiota" as used herein refers to the community of microorganisms that occur (sustainably or transiently) in and on a subject (e.g., a human subject), including eukaryotes, archaea, bacteria, and viruses (including bacterial viruses, e.g. phage). In some embodiments, microbiota specifically refers to the microbial community in a niche.

The present embodiments encompass the recognition that microbial signatures can be relied upon as proxy for microbiome composition and/or activity. Microbial signatures comprise data points that are indicators of microbiome composition and/or activity. Thus, according to the present invention, changes in microbiomes can be detected and/or analyzed through detection of one or more features of microbial signatures.

As used herein, the term "metabolome" refers to the collection of metabolites present in the individual. The human metabolome encompasses native small molecules (natively biosynthesizeable, non-polymeric compounds) that are participants in general metabolic reactions and that are required for the maintenance, growth and normal function of a cell. Typically, metabolites are small molecule compounds, such as substrates for enzymes of metabolic pathways, intermediates of such pathways or the products obtained by a metabolic pathway.

As used herein, the term "microbiota-derived metabolites" refers to any metabolites originating from the microbiota, or present within organisms in the microbiota, and that can therefore, if present, be detected from a subject's sample. As a complex ecosystem, the microbiota contains a large number of different organisms, each of which produce metabolites as part of their intracellular metabolic processes.

The analysis of the databases according to some embodiments of the present invention comprises executing a machine learning or statistical modeling procedure.

As used herein the term "machine learning" refers to a procedure embodied as a computer program configured to induce patterns, regularities, or rules from previously collected data to develop an appropriate response to future data, or describe the data in some meaningful way.

Use of machine learning is particularly, but not exclusively, advantageous when the database includes multidimensional entries.

The group and subject databases can be used as a training set from which the machine learning procedure can extract parameters that best describe the dataset. Once the parameters are extracted, they can be used to predict the response for the selected food.

In machine learning, information can be acquired via supervised learning or unsupervised learning. In some embodiments of the invention the machine learning procedure comprises, or is, a supervised learning procedure. In supervised learning, global or local goal functions are used to optimize the structure of the learning system. In other words, in supervised learning there is a desired response, which is used by the system to guide the learning.

In some embodiments of the invention the machine learning procedure comprises, or is, an unsupervised learning procedure. In unsupervised learning there are typically no goal functions. In particular, the learning system is not provided with a set of rules. One form of unsupervised, learning according to some embodiments of the present invention, is unsupervised clustering in which the data objects are not class labelled, a priori.

Representative examples of "machine learning" procedures suitable for the present embodiments, including, without limitation, clustering, association rule algorithms, feature evaluation algorithms, subset selection algorithms, support vector machines, classification rules, cost-sensitive classifiers, vote algorithms, stacking algorithms, Bayesian networks, decision trees, neural networks, instance-based algorithms, linear modelling algorithms, k-nearest neighbors analysis, ensemble learning algorithms, probabilistic models, graphical models, regression methods, gradient ascent methods, singular value decomposition methods and principle component analysis. Among neural network models, the self-organizing map and adaptive resonance theory are commonly used unsupervised learning algorithms. The adaptive resonance theory model allows the number of clusters to vary with problem size and lets the user control the degree of similarity between members of the same clusters by means of a user- defined constant called the vigilance parameter.

An *in silico* method is an expression meaning "performed on computer or via computer simulation" in reference to biological experiments.

The "relative abundance" of a particular bacterium in a bacterial community, or equivalently, of a particular nucleic acid sequence in a collection/population of metagenomic sequences, is defined as the proportion of the overall population covered by this specific bacterium or nucleic acid sequence. Thus, it is not a measure of absolute abundance, but rather a relative one in comparison to the other bacteria/nucleic acid sequences in the population. As such, it takes a value between 0 and 100%, or in fractional terms, 0 and 1.

It is one of the objects of the present invention to provide a method for predicting a response to different dietary fibres from nucleic acid sequences, based on the analysis of the fermentation or metabolic capabilities of a subject's gut microbiota assessed by its short chain fatty acid (SCFA) production rate in response to said different dietary fibres, the method being applied to abundance data of specific microbiota-derived nucleic acids obtained from prior collected stabilized biological sample of said subject, the method comprising the steps of:

- isolating and extracting said specific microbiota-derived nucleic acids as well as microbiota-derived metabolites from said stabilized biological sample, wherein the specific sequences of said specific microbiota-derived nucleic acids are identified from a training database containing metagenomics sequencing data from healthy subjects, or subjects suffering from a particular disease, paired with *in vitro* measured data pertaining to the production rates of SCFAs and other fermentation metabolites from different dietary fibers, selected from the list comprising propionate, butyrate, acetate, lactate, formate, succinate, iso-butyrate, valerate and iso-valerate;

- determining and quantifying the relative abundance of said specific microbiota-derived nucleic acids from said stabilized biological sample to generate a first input, wherein said specific microbiota-derived nucleic acids consist in gene sequences specific to glycoside and/or polysaccharide hydrolases capable of hydrolyzing or cleaving complex polysaccharides of said different dietary fibres, as well as gene sequences specific to key enzymes from bacterial acetate, propionate and butyrate production pathways and/or enzymes responsible for the degradation of said different dietary fibers;

- applying a machine learning algorithm to predict from these specific microbiota-derived nucleic acid sequences the production rate of SCFAs and other fermentation metabolites in response to challenge with different dietary fibers so as to obtain a response to said different dietary fibres.

Contrary to the prior art, the stabilized biological sample is not to be treated with dietary fibres in the present invention. On the contrary, the invention seeks to predict the fermentation capability of an individual (i.e. their capacity to produce certain metabolites from certain dietary fibre inputs) from nucleic acids alone. This is possible because of the machine learning algorithms which are trained on a database containing analyses of stool samples that were challenged with dietary fibres and for which the metabolites were measured experimentally, in addition to their nucleic acids being sequenced. The machine learning algorithms are trained to predict these experimental fiber challenge results from the nucleic acid sequences, such that they can be simply applied to other, new samples which only have to be sequenced and therefore can be preserved in a stabilizing buffer at room temperature for a longer period of time (e.g. to allow for mailing/shipping).

Thus, the invention concerns a method of predicting from nucleic acids in a new sample using algorithms that were trained on the data that include incubation with specific fibers (and therefore can only predict responses to these specific fibers), which include analyses of the metabolites mentioned above.

By the way the present invention differs significantly from WO 2019/046372 AI (UNIV MICHIGAN REGENTS [US]), since boosting butyrate production with dietary fibers requires knowledge of the microbiota composition, due to the fact that not all fibers are well fermented by a given individual's microbiota into SCFAs, while others are.

Preferably, the training database comprises data classified according to a predetermined set of classification groups, wherein said analysis comprises classifying the subject according to a specific type or phenotype.

According to an embodiment of the invention, the software further comprises a set of algorithms for linking the data associated with the subject's microbiome to a certain medical condition, physical condition, or likely responsiveness to a certain therapy.

In particular, the method of the invention further comprises steps for stratifying the subject to different treatment plans, including personalized dietary recommendations or other therapeutic strategies aiming to improve the subject's microbiota's fermentation capabilities so as to build the subject's microbiome profile. According to an embodiment of the invention, the personalized dietary recommendations are formulated by identifying the dietary fibers inputs that result in the highest and lowest production of metabolites of interest by the subject's microbiota so as to establish personal care product to the subject.

According to another embodiment of the invention, the therapeutic strategies aiming to improve the subject's microbiota's fermentation capabilities comprise one or more prebiotics, one or more probiotics, one or more antibiotics, or any other drug for therapeutic treatment disease or disorder with a metabolic and/or immunological and/or inflammatory involvement.

In the context of the invention, different treatment plans include predisposition to a disease or disorder with a metabolic and/or immunological and/or inflammatory involvement which is selected from the group comprising obesity, metabolic syndrome or diseases, diabetes mellitus such as Type-2 diabetes, insulin-deficiency related disorders, insulin-resistance related disorders, intestinal gluconeogenesis disorders, inflammation disorders, inflammatory Bowel disease, systemic or local inflammation in the context of neurodegeneration, depression or anxiety disorders, food intolerance, diarrhea, constipation, colitis and enteritis, allergies, rheumatoid arthritis, and cancer immunotherapy applications.

Preferably, the subject's biological sample is selected from the group comprising: a rectal swab, a fecal sample, a biopsy or a mucosal layer sample. More preferably the subject's biological sample is stool sample. The sample is also safe for shipping.

Advantageously, the *in vitro* method of the invention allows the determination and quantification of the relative abundance of different bacterial nucleic acids which is performed by either DNA sequencing specific marker genes including 16S rRNA, shotgun metagenomics DNA sequencing, PCR or qPCR of specific genes, or method for nucleic acid quantification including capillary electrophoresis.

According to an embodiment of the invention, the specific metabolites of interest comprise short chain fatty acids assessment consisting of short chain fatty acids propionate and butyrate. However further specific metabolites of interest comprise acetate, lactate, formate, succinate, iso-butyrate, valerate and iso-valerate.

According to another embodiment, the other enzymes responsible for the degradation of said different dietary fibres are selected from the group comprising bacterial, archaeal, or fungal organisms originated from the subject's gut microbiota. Depending on the individual, the collective ability of their microbiota to degrade specific fibres can come from the interplay of several organisms; for example, a certain enzyme from commensal fungi in the microbiota can degrade a fibre into its constituent oligosaccharides or monosaccharides (e.g. pectin into galacturonic acid), which are subsequently fermented by bacterial organisms into short chain fatty acids.

Preferably the machine-learning algorithms comprise a supervised learning procedure. More preferably, the machine learning algorithms comprise at least one procedure selected from the group consisting of clustering, support vector machine, linear modeling, k-nearest neighbors' analysis, decision tree learning, ensemble learning procedure, neural networks, probabilistic model, graphical model, Bayesian network, and association rule learning.

The machine learning algorithms allowing user to identify an individual's fermentation capability consist of several layers. The first layer consists of individual classifiers or regressors that predict the production rate of a specific metabolite (e.g. an SCFA) in response to a specific dietary fiber, using the relative abundances of specific nucleic acid sequences as inputs. These individual classifiers or regressors would be trained on the database mentioned above. This layer of classifiers or regressors alone can describe an individual's fermentation capability. The results from this layer can then be fed into a second layer that combines the results of these specific metabolites in order to identify the optimal mixture or combination of dietary fibers that would result in a desired metabolite production profile (e.g. maximizing butyrate production while minimizing propionate production).

In the context of the invention, the different dietary fibres are complex polysaccharides or other dietary ingredients selected from the group comprising: inulins, fructo-oligosaccharide, resistant starch, lignin, tannin, cellulose, hemicelluloses (including xyloglucans, glucuronoxylans, arabinoglucuronoxylans, arabinoxylans, heteroxylans, mannans, and beta-glucans), psyllium, polydextrose, chitin, chitosan, pectins (including homogalacturonans/polygalacturonans, and rhamnogalacturonans I and II), arabinan, and konnyaku / konjac (glucomannan).

According to a preferred embodiment of the invention, the method for predicting a response to different dietary fibres is applied on subject's sample stool using a wipe and treating the wipe with a nucleic acid stabilization solution (e.g. ethanol, RNAlater, etc.). Once stabilized, the nucleic acids in the stool are stable at room temperature and can be mailed. The sample is also safe for shipping.

Mucosal layer sample can be collected by a biopsy during an endoscopic procedure, or as a fresh stool sample in the clinic.

Microbiota-derived nucleic acids are extracted from the sample.

The method then determines and/or quantifies the relative abundance of different nucleic acid sequences from the stool or mucosa sample, either by DNA sequencing specific marker genes (e.g. 16S rRNA), shotgun metagenomic DNA sequencing, PCR or qPCR (quantitative PCR) of specific genes, or another method for nucleic acid quantification (e.g. capillary electrophoresis).

DNA sequencing (initially 16S rRNA amplicon sequencing) implies creation of a set of specific PCR primers for the key features namely the gene sequences specific to glycoside and/or polysaccharide hydrolases capable of hydrolyzing or cleaving complex polysaccharides of said different dietary fibres, as well as gene sequences specific to key enzymes from bacterial acetate, propionate and butyrate production pathways. However, method for quantifying the relative abundance of specific sequences using capillary electrophoresis can also be used as well as any other method known to the skilled person for of nucleic acid quantification that has sufficient resolution.

The next step is to input these nucleic acid quantification or sequencing data into a software consisting of a statistical model or machine-learning algorithm that predicts the fermentation capabilities of the microbiota. More specifically, the software predicts the ability of the microbiota to produce specific metabolites by the microbiota from different dietary inputs. Key metabolites are the Short Chain Fatty acids propionate and butyrate. This model was trained on/developed from a database. Additional metabolites of interest include acetate, lactate, formate, succinate, iso-butyrate, valerate, and iso-valerate.

In particular, the software or algorithm takes as input the relative abundance of specific nucleic acid sequences; Scores the microbiota's ability to produce specific metabolites by predicting either a production rate, or a relative measure of production capability (e.g. high VS low compared to a sampled population, z-score compared to a sampled population) of each metabolite in question in response to a specific set of dietary inputs. Specific dietary inputs include individual types of dietary fibres and complex polysaccharides. This is achieved using a statistical or machine learning model that acts on the inputed features. Additionally, the model may instead return a single variable, namely a specific fermentation 'type' (where the continuum of different fermentation capabilities observed in the human population are discretized into specific types/phenotypes, e.g. Type 1, Type 2, Type 3, etc.). In addition the model may compute a score from the relevant features using a weighted, linear or non-linear combination of input features and a hierarchical combination of one or more statistical classifiers or regressors.

The above collected information is used in the method to stratify the user/patient to different treatment plans, which may include personalized dietary recommendations or other therapeutic strategies aiming to improve subject's microbiota's fermentation capabilities (e.g. treatment with prebiotics, probiotics, synbiotics (i.e. prebiotics + probiotics), or faecal microbiota transplantation).

Advantageously, the method allows to identify the dietary inputs that result in the highest and lowest production of metabolites of interest by the microbiota and use these results to formulate personalized dietary/nutritional recommendations. This can be in the form of specific supplements containing the dietary inputs in question, or as general recommendations of different dietary ingredients such as specific fruits, vegetables, grains, etc. aiming to result in the production of specific metabolites. The latter general recommendations can be computed from look-up tables containing the quantities of each specific dietary input present in different ingredients.

Construction of the training database:

Collect stool sample, stool biopsy or mucosal biopsy.

Process the sample under anaerobic conditions, which includes homogenizing the sample into a slurry and diluting it to a concentration that preserves the metabolic activity of the microbiota but allows for the conducting of all necessary downstream fermentation experiments.

Take a baseline aliquot of the sample for nucleic acid quantification.

Take a baseline aliquot for metabolite quantification (as described above).

Incubate individual aliquots of the sample under anaerobic conditions and at 37 degrees Celsius (temperature of the human body). Aliquots are incubated in the presence of specific dietary ingredients or spike-ins, under anaerobic conditions and at 37 degrees Celsius (temperature of the human body). Each different spike-in (including one or more controls with no spike-ins) is referred to as a specific 'spike-in condition' from this point, for clarity. Sample from each spike-in condition at different time intervals (e.g. hourly).

Quantify the presence/absence or concentration of metabolites of interest from each timepoint of interest.

Combine these data into specific production rates or production capabilities (e.g. binary capability such as able or not able to produce the metabolite).

Store the nucleic acid quantification data of the initial sample (which constitutes the information about the microbiota composition), and the sample microbiota's production capability of different metabolites in response to different spike-ins, in a database.

According to a particular embodiment, the invention provides an *in vitro* method for predicting a response to different dietary fibres based on the analysis and measuring of the fermentation or metabolic capabilities of a subject's gut microbiota assessed by its short chain fatty acid (SCFA) production rate in response to said different dietary fibres, the method being applied to prior collected stabilized biological sample of said subject, the method comprising the steps of:

- isolating and extracting microbiota-derived nucleic acids as well as microbiota-derived metabolites from said stabilized biological sample;
- determining and quantifying the relative abundance of said microbiota-derived metabolites and of said different microbiota-derived nucleic acids from said stabilized biological sample to generate a first input, wherein said different microbiota-derived nucleic acids consist in gene sequences specific to glycoside and/or polysaccharide hydrolases capable of hydrolyzing or cleaving complex polysaccharides of said different dietary fibres, as well as gene sequences specific to key enzymes from bacterial acetate, propionate and butyrate production pathways;
- applying said first input into a training database having data pertaining to responses on the fermentation capabilities from the microbiota of other healthy subjects, or subjects suffering from a particular disease, and analyzing said resulting database into a software consisting of a statistical model and/or machine-learning algorithm configured to predict the ability of said subject's gut microbiota to produce specific metabolites of interest from said different dietary fibres, wherein said specific metabolites of interest comprise short chain fatty acids assessment and wherein said machine-learning algorithm extracts parameters from said resulting database to predict the response of said subject to said different dietary fibres.

The mentioned technical features are as described above.

According to still another embodiment, the invention provides an in vitro method for predicting a response to different dietary fibres based on the analysis and measuring of the fermentation or metabolic capabilities of a subject's gut microbiota assessed by its short chain fatty acid (SCFA) production rate in response to said different dietary fibres, the method being applied to prior collected stabilized biological sample of said subject, the method comprising the steps of:

- cultivating said stabilized biological sample in the presence of said different dietary fibers in order to detect the fermentation or metabolic capabilities of said subject's gut microbiota;
- isolating and extracting microbiota-derived nucleic acids as well as microbiota-derived metabolites from said stabilized biological sample;
- determining and quantifying the relative abundance of said microbiota-derived metabolites and of said different microbiota-derived nucleic acids from said stabilized biological sample to generate a first input, wherein said different microbiota-derived nucleic acids consist in gene sequences specific to glycoside and/or polysaccharide hydrolases capable of hydrolyzing or cleaving complex polysaccharides of said different dietary fibres, as well as gene sequences specific to key enzymes from bacterial acetate, propionate and butyrate production pathways;
- applying said first input into a training database having data pertaining to responses on the fermentation capabilities from the microbiota of other healthy subjects, or subjects suffering from a particular disease, and analyzing said resulting database into a software consisting of a statistical model and/or machine-learning algorithm configured to predict the ability of said subject's gut microbiota to produce specific metabolites of interest from said different dietary fibres, wherein said specific metabolites of interest comprise short chain fatty acids assessment selected from the list comprising propionate, butyrate, acetate, lactate, formate, succinate, iso-butyrate, valerate and iso-valerate and wherein said machine-learning algorithm extracts parameters from said resulting database to predict the response of said subject to said different dietary fibres.

The mentioned technical features are as described above.

Another object of the present invention is to provide a computer software product, comprising a computer-readable medium in which program instructions are stored, which instructions, when read by a data processor, cause the data processor to receive data collected from a subject's sample, and to execute the *in vitro* method according to the invention.

Computer software or programs implementing the method of the present embodiments can commonly be distributed to users on a distribution medium such as, but not limited to, CD-ROMs or flash memory media. From the distribution medium, the computer programs can be copied to a hard disk or a similar intermediate storage medium. In some embodiments of the present invention, computer programs implementing the method of the present embodiments can be distributed to users by allowing the user to download the programs from a remote location, via a communication network, e.g., the internet. The computer programs can be run by loading the computer instructions either from their distribution medium or their intermediate storage medium into the execution memory of the computer, configuring the computer to act in accordance with the method of this invention. All these operations are well-known to those skilled in the art of computer systems.

A further object of the invention is to provide an apparatus for predicting a response of a subject to different dietary fibres, the apparatus comprising: a user interface configured to receive a subject's sample; a module for extracting microbiota-derived nucleic acids from said subject's sample and for quantifying and/or sequencing said nucleic acids; and a data processor having a computer-readable medium storing the computer software product of the invention.

In summary, the apparatus:

1) accepts a subject's sample at the User Interface,
2) extracts microbiota-derived nucleic acids and quantifies/sequences them;
3) runs the software using the data processor.

The apparatus of the invention, is suitable for a multidimensional analysis procedure optionally and preferably comprising a machine learning procedure, such as one or more of the procedures described above. The purpose of the analysis is to determine patterns in the data that allow finding similarities among different entries in the database. For example, the analysis can include defining classification groups into which the database entries can be classified, and classifying the database entries according to the classification groups. Subsequently, entries can be labelled according to their classification. The multidimensional analysis can additionally or alternatively comprise the construction of a decision tree. Subsequently, the database can be updated to include the constructed decision tree. The multidimensional analysis can additionally or alternatively comprise extraction association patterns and/or association rules among features in the database. Subsequently, the database can be updated to include the association patterns and/or association rules. The multidimensional analysis can additionally or alternatively comprise ranking of features in the database, e.g., using feature evaluation algorithm. Subsequently, the database can be updated to include the ranks. The multidimensional analysis can additionally or alternatively comprise constructing a Bayesian network from at least a portion of the data of the database. Subsequently, the database can be updated to include the constructed Bayesian network. Other types of analyses are also contemplated.

The term "decision tree" refers to any type of tree-based learning algorithms, including, but not limited to, model trees, classification trees, and regression trees.

According to a preferred embodiment, the apparatus of the invention, further comprises a PCR kit for sequencing and quantifying the relative abundance of said specific microbiota-derived nucleic acids identified from a training database, said PCR kit comprising primers specific for nucleic acid sequences that are identified from the training database as most predictive of fiber response, and optionally further includes primers specific for sequences from the genes and metabolic pathways specific to key enzymes from bacterial acetate, propionate and butyrate production pathways, as well as other enzymes responsible for the degradation of said different dietary fibres.

These specific nucleic acid sequencing primers can then be refined from publicly available reference genomes to ensure that they will retain specificity across different individuals who may contain bacterial strains that vary slightly in these specific sequences due to minor mutations between strains. The kit allows for sequencing of these markers specifically, allowing an efficient quantification of the relative abundance of these markers in the individual's gut microbiota, and provide the exact inputs required for the machine learning algorithms to make accurate predictions.

According to a preferred embodiment, the PCR kit further includes primers specific for sequences from the genes and metabolic pathways specific to key enzymes from bacterial acetate, propionate and butyrate production pathways, as well as enzymes responsible for the degradation of specific fibers. These latter enzymes can be bacterial, archaeal, or fungal, and thus can be drawn from any part of the gut microbiota.

As noted above, certain fungi can contain enzymes that can degrade dietary fiber, and therefore they also contribute to an individual's fermentation capability. For example, pectinases can sometimes be found not only in bacteria but in fungi, and specifically degrade pectin into oligo- and monosaccharides that can further be fermented by the bacterial microbiota. In contrast, fructanases that degrade fructans such as inulin are typically found in bacteria.

As described above, the invention aims at determining the fermentation or metabolic capabilities of an individual's gut microbiota from for example stool nucleic acid sequencing (metagenomic DNA or metatranscriptomic RNA), as well as from analysis of stool metabolomics/metabolite profiles. It allows a user or patient or subject to quantify the ability of their gut microbiota to ferment different polysaccharides or dietary compounds to produce specific metabolites of physiological or clinical interest. Furthermore, the invention allows the user or patient to determine which polysaccharide(s) or dietary compound(s) could be consumed as a supplement in order to increase or decrease the production of specific metabolites by their gut microbiota.

Thus, the invention constitutes both a diagnostic/prognostic and a framework for formulating personalised dietary strategies towards specific clinical outcomes. This framework includes pairing the diagnostic with specific dietary fibres or compounds (in powdered form, or integrated into a functional food). Physiological applications include minimising inflammation, increasing intestinal gluconeogenesis, or maximising any other health benefits that can be conferred by gut microbiota-derived metabolites generated through fermentation processes, including but not limited to Short Chain Fatty Acids (SCFAs). Clinical applications include but are not limited to improving efficacy of cancer immunotherapy; decreasing intestinal inflammation in the context of Inflammatory Bowel Disease or similar ailments; decreasing systemic or local inflammation in the context of neurodegeneration, rheumatoid arthritis, depression or anxiety; improving metabolic health in the context of metabolic disease or obesity; and prevention of Type-2 diabetes. Additionally, an apparatus or device can be created that encompasses both the sequencing/nucleic acid detection/analytical chemistry capability, and the software for prediction of microbiota metabolic capabilities and formulation of resulting personalised dietary recommendations.

The software that formulates these predictions consists of statistical and/or machine learning models trained on a database of experimental data consisting of experimental measurements from the stool microbiota of volunteers. More specifically, for each volunteer's microbiota, the database contains metagenomic DNA and/or metatranscriptomic RNA sequencing and/or metabolomics data of the raw stool, paired to experimental measurements obtained anaerobically from the live microbiota in the sample. These experimental measurements include, but are not necessarily limited to, the time-resolved measurement of SCFAs and intermediate fermentation metabolites produced over time in response to particularly chemical spike-ins.

These chemical spike-ins refer to specific dietary fibres, complex polysaccharides or other dietary ingredients, but can also include drugs/medications in combination with the aforementioned chemicals. In particular, these spike-ins include a panel of dietary fibres that span the dominant categories of dietary fibres, including (but not limited to):

- inulins
- fructo-oligosaccharide
- resistant starch
- lignin
- tanin
- cellulose
- hemicelluloses (including xyloglucans, glucuronoxylans, arabinoglucuronoxylans, arabinoxylans, heteroxylans, mannans, and beta-glucans)
- psyllium
- polydextrose
- chitin
- chitosan
- pectins (including homogalacturonans/polygalacturonans, and rhamnogalacturonans I and II)
- arabinan
- konnyaku / konjac (glucomannan).

This database can also take the form of specific bacterial strains (and accompanying marker gene sequences) and their experimentally measured metabolic capabilities.

Key features used by the prediction algorithms include:

- glycoside hydrolases and polysaccharide utilisation loci specific for hydrolysing or cleaving specific complex polysaccharides or dietary fibres
- marker genes of butyrate production pathways, including butyryl-coenzyme A (CoA):acetate CoA-transferase, butyrate kinase and phosphotransbutyrylase
- marker genes of propionate production pathways, including methylmalonyl-CoA decarboxylase subunit alpha (mmdA) (marker of succinate pathway), lactoyl-CoA dehydratase subunit alpha (lcdA) (marker of acrylate pathway), aldehyde dehydrogenase (pduP).
- marker genes of acetate production pathways, including acetyl-CoA-Synthase and Carbon monoxide dehydrogenase (markers of the Wood-Ljungdahl pathway).

They may also include specific nucleic acid markers for specific bacterial strains known to harbour the enzymes mentioned above.

It is an advantage of the present invention to predict an individual's SCFA production capability from different dietary ingredients using only specific metagenomic or metatranscriptomic sequencing features, or metabolomic/metabolite features from stool; specifically, surprisingly the invention allows to predict the microbiota's metabolic response to foods and its production of specific metabolites rather than the individual or patient's response (e.g. post-prandial glucose response)

Advantageously the invention allows to make predictions using nucleic acid sequencing from a stool collection mailer kit, where the nucleic acids in the stool are stabilized. It is to be noted that the prior art experimental protocols for quantifying a person's microbiota's metabolic capabilities involve anaerobic culturing of live stool microbiota, which requires a fresh stool sample (treated anaerobically within hours of passage) and is therefore laborious, costly, and logistically highly limiting (particularly for clinical applications).

Interestingly, the invention also allows to select from a list of dietary supplements the specific supplement that will maximize the production of a given SCFA or metabolite based on an individual's microbiota.

Besides the invention can be applied to a diagnostic tool or process in the context of treating disease, whether maximizing butyrate production in patients suffering from Inflammatory Bowel Disease, neurodegeneration, rheumatoid arthritis, depression or anxiety; maximizing butyrate and propionate production in obese, pre-diabetic/diabetic (type-2), or metabolic syndrome patients; or maximizing propionate production in patients undergoing cancer immunotherapy.

As shown in the examples of the present invention, Applicants have used an *ex vivo* setup to measure the inter-individual differences in gut microbial SCFA production in response to different dietary fiber inputs. Applicants have shown that there are significant differences in the MMPs of different individuals, i.e. differences in the capacity of their microbiota to ferment a given fiber substrate into a given SCFA. Moreover, Applicants showed that MMP could to a certain extent be predicted from stool microbiota community composition, to a greater extent than can raw stool SCFA content.

In addition, Applicants showed that the dominant features of an individual's MMP are relatively stable through time, consistent with the fact that an individual's MMP is related to their microbiota community composition. Though Applicants did not study such cases, it is likely that treatment with broad-spectrum antibiotics or other extreme perturbations to the microbiota would have significant impact to an individual's MMP, with concomitant implications to their physiology.

In order to explore the relationship between Applicants' *ex vivo* results and the implications for *in vivo* SCFA production and absorption, Applicants sought to develop a phenomenological model of the process. Applicants found that quantities of absorbed SCFAs mirrored the quantities produced in the different parameter regimes considered, though the absolute amounts varied significantly as a function of the epithelial absorption rate constants used in their model. This highlights the critical importance of this variable in understanding the relationship between stool SCFA quantities and in vivo production and absorption for clinical applications. It is therefore of great importance to the field to obtain accurate measurements of these rate constants in addition to quantitative descriptions of their behavior and the underlying kinetics of absorption at different concentrations of SCFAs and in different regions of the gut.

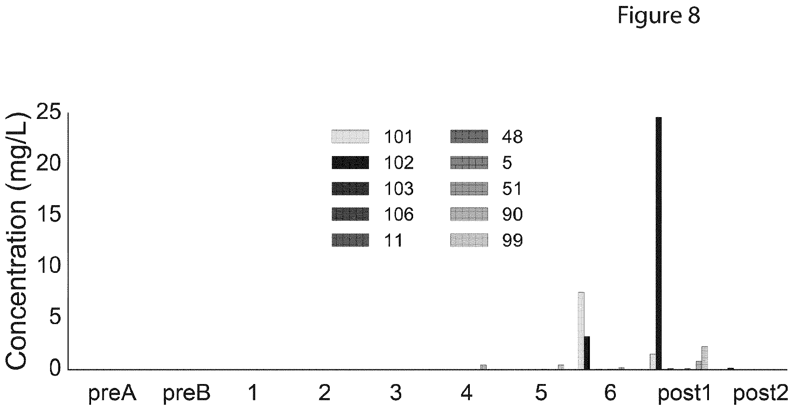

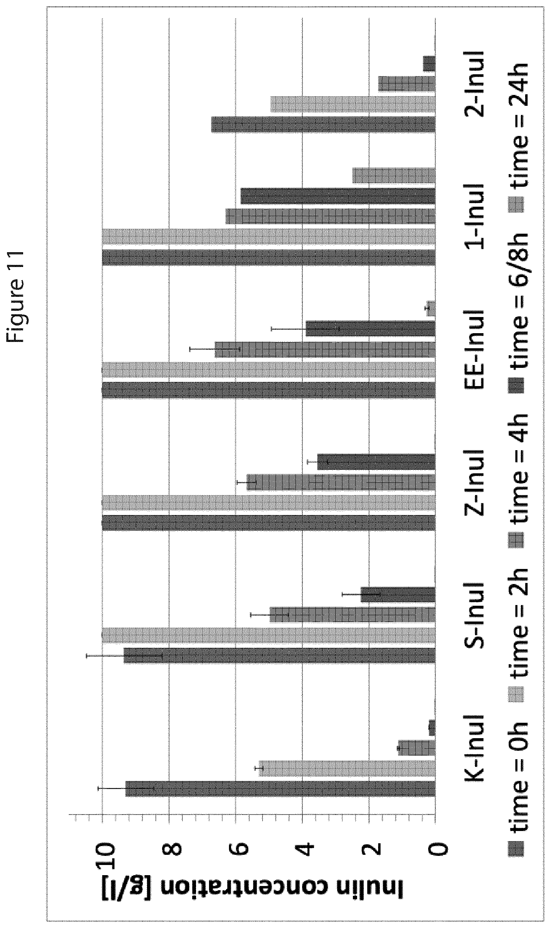

Applicants' model also showed that increased colonic transit time results in significantly greater quantities of absorbed SCFAs while these remain at appreciable concentrations in the gut, or that there remains substrate to ferment. In Applicants' *ex vivo* experiments, as well as the *in vivo* inulin supplementation experiments used in Fig. 8, the microbiota was given a highly accessible, powdered form of inulin as a substrate. Despite the fact that both *ex vivo* and *in vivo*, the majority of inulin is fermented by the microbiota, within 24h or by the time of passage, respectively, Applicants' *ex vivo* measurements indicate that this process occurs on the order of 12-24h to occur in most participants at the considered input concentration (10g/L) (Fig. 11). Thus, it is likely that a less accessible form of fibre input (e.g. in the form of raw chicory root in the case of inulin) would take longer to ferment, in which case colonic transit time is an important variable in determining the quantities of SCFAs produced. Moreover, dietary fiber supplementation therapeutic strategies aiming to improve quantities of SCFAs produced would benefit from highly accessible forms of these fibers (purified powder), rather than grains, fruits and vegetables high in these quantities, which are likely to take longer for the microbiota to ferment and therefore may result in a larger quantity of excreted, unfermented substrate.

Applicants' model suggests a high degree of robustness to the association between differences in MMPs and quantities of absorbed SCFAs across different individuals, despite the fact that varying the absorption rate constant had significant impact on both absorbed and excreted SCFA quantities. Importantly, depending on the actual magnitude of the absorption rate constant, the relationship between stool SCFA concentration and *in vivo* production and absorption can be completely non-informative. Stool SCFA concentration is related to the differential between production and absorption, the colonic transit time, and whether or not the entire fermentable substrate was consumed. For example, in a regime where production rate significantly exceeds absorption rate (as in both sets of absorption rate constant parameters considered in this study), stool SCFA is a function of the differential between the transit time ($\tau$) and the time to depletion of the fermentable substrate (t1). If $\tau$ > t1, the stool SCFA concentration will mostly be a function of the time during which the stool was transiting but no further SCFAs were being produced (and luminal SCFAs were merely being absorbed). This is an important consideration that suggests that stool SCFA quantities may not be the relevant quantity of interest in the absence of knowledge of these other variables. Put differently, a lack of association between stool SCFA concentration and a clinical outcome variable is not necessarily indicative of a lack of involvement of microbial SCFAs in the disease process.

Taken together, Applicants' data indicate that a quantitative understanding of a patient's MMP can inform personalized dietary supplementation strategies that aim to increase the production and resulting absorption of specific SCFAs in a patient's gut. More broadly, they suggest a framework for modulating SCFA production in a patient through two separate but complimentary means: modification of dietary inputs as a function of their existing MMP, and modification of the underlying community composition of their microbiota towards a given MMP of interest.

Those skilled in the art will appreciate that the invention described herein is susceptible to variations and modifications other than those specifically described. The invention also includes all of the steps, features, compositions and compounds referred to or indicated in this specification, individually or collectively, and any and all combinations or any two or more of said steps or features. The present disclosure is therefore to be considered as in all aspects illustrated and not restrictive, the scope of the invention being indicated by the appended Claims, and all changes which come within the meaning and range of equivalency are intended to be embraced therein.

The foregoing description will be more fully understood with reference to the following Examples. Such Examples, are, however, exemplary of methods of practising the present invention and are not intended to limit the scope of the invention.

**Examples**

**Materials and Methods:**

Human participants

Healthy human participants were consented to participate in this study.

Stool sample processing and *ex vivo* setup

Fresh stool samples were collected and weighed before being transferred to anaerobic conditions, in which they were diluted in reduced PBS containing 0.1% L-Cysteine to a ratio of 1g/5ml. Samples were homogenized into a slurry before being aliquoted into 96-well plates. Samples of the unaltered slurry were taken for a baseline sample, after which inulin, pectin, and cellulose were added from stock solutions to final slurry concentrations of 10g/L, 5g/L and 20g/L, respectively. Concentrations of inulin and pectin were determined based on the maximum stock concentration we were able to obtain in which the dietary fiber was fully dissolved. Four conditions were measured: inulin, pectin, cellulose and control (no spike-in). The samples were incubated anaerobically at 37°C, and two biological replicates (two different wells in the 96-well plates) were collected at each time point. Samples were collected after 2 and 4 hours from all participants and sent out for SCFA quantification on a GC-FID. Linear production rate was measured between 2h and 4h because this allowed Applicants maximum accuracy in measuring the time interval without introducing artefacts due to delays between conditions introduced during setup. Results obtained from slurry in large flasks on a shaker were in good agreement with data obtained in a 96-well plate format.

Measurements of gas produced in the *ex vivo* set up

Adaptation from *ex vivo* set up:

Stool sample were prepared, homogenized and different conditions are prepared as in the traditional ex vivo experiment. Once the conditions mastermixes (control no fiber, with 10g/L inulin, 5g/L pectin or 20g/L cellulose) are prepared, they are transferred to a vinyl anaerobic chamber filled with 100% N2, with no detectable amounts of CO2 and H2. For each participant, 2mL of the final fecal slurry of each condition was added in triplicates to 60mL glass serum bottles (Supelco, Bellefonte PA). The total of 12 bottles per participant were sealed in the same chamber using magnetic crimp seals with PTFE/silicone septa (Supelco, Bellefonte PA), and incubated at 37°C for 24h.

Gas measurements:

Concentrations of headspace gases were determined using gas chromatography. Applicants used a Shimadzu GC-2014 gas chromatography (GC) configured with a packed column (Carboxen-1000, 5' x 1/8" (Supelco, Bellefonte PA)) held at 140°C, argon gas carrier fas, thermal conductivity and methanizer-flame ionization detectors. Subsamples of the headspace (0.20 cm3) at the laboratory temperature, ca. 23°C) from each serum bottle were taken via a gas-tight syringe and infected onto the column. Gas concentrations were determined by comparing the partial pressures of samples and standards with known concentrations.

Accuracy of the analyses, evaluated from standards, was ±5%.

Sample collection for other purpose:

1mL of the fecal slurry was taken out from each serum bottle at the end of this experiment and GC measurements for SCFA analysis.

Short-chain Fatty acid measurements

Gas chromatography analysis:

Chromatographic analysis was carried out using an Agilent 7890B system with a flame ionization detector (FID) (Agilent Technologies, Santa Clara, CA). A high resolution gas chromatography capillary column 30m x 0.25 mm coated with 0.25 $\mu$m film thickness was used (DB-FFAP) for the volatile acids (Agilent Technologies) and a high resolution gas chromatography capillary column 30m x 0.25 mm coated with 0.50 $\mu$m film thickness was used (DB-FFAP) for the nonvolatile acids. Nitrogen was used as the carrier gas. The oven temperature was 145°C and the FID and injection port was set to 225°C. The injected sample volume was 1$\mu$L and the runtime for each analysis was 12 minutes. Chromatograms and data integration was carried out using the OpenLab ChemStation software (Agilent Technologies)
Standard Solutions:
A volatile acid mix containing 10mM of acetic, propionic, isobutyric, butyric, isovaleric, valeric, isocaproic, caproic, and heptanoic acids was used (Supelco CRM46975, Bellefonte, PA). A standard stock solution containing 1% 2-methyl pentanoic acid (Sigma-Aldrich St. Louis, MO) was prepared as an internal standard control for the volatile acid extractions. A non-volatile acid mix containing 10mM of pyruvic and lactic and 5mM of oxalacetic, oxalic, methy malonic, malonic, fumaric, and succinic was used (Supelco, 46985-U, Bellefonte, PA). A standard stock solution containing 50mM benzoic acid (sigma-Aldrich St. Louis, MO) was prepared as an internal standard control for the nonvolatile acid extractions.

Sample preparation:

The samples were kept frozen at -80°C until analysis. The samples were removed from the freezer and allowed to thaw. A sample of the raw fecal material was transferred to a 2mL tube, the weight of the fecal material was determined and 1.5 mL of HPLC water was added to each sample. The samples were vortexed for 5 minutes until the material was homogenized. The pH of raw fecal suspension and the thawed fecal slurry samples was adjusted to 2-3 with 50% sulfuric acid. The acidified samples were kept at room temperature and vortexed for 10 minutes. The samples were centrifuged for 10 minutes at 5000g. 1000uL of the clear supernatant was transferred into a glass tube with a PTFE faced rubber lined screw cap for further processing. 50uL of the internal standard (1% 2-methyl pentanoic acid solution) and 1ml of ethyl ether anhydrous were added to the volatile samples. The tubes were mixed end over end for 10 minutes and then centrifuged at 2000g for 1 minute. The upper ether layer was transferred to an Agilent sampling vial for analysis. For the nonvolatile extraction, 50uL of the internal standard (50mM benzoic acid solution) and 1mL boron trifluoride-methanol solution (Sigma-Aldrich St.Louis, MO) were added to each tube containing 1000uL of the clear supernatant. These tubes were incubated overnight at room temperature. 2mL of water and 1mL of chloroform were added to each tube. The tubes were mixed end over end for 10 minutes then centrifuges at 2000g for 1 minute. The lower chloroform layer was transferred to an Agilent sampling vial for analysis.

Quantification of Acids:

1ml of each of the standard mixes were used and processed as described for the samples. The retention times and peak heights of the acids in the standard mix were used as references for the sample unknowns. These acids were identified by their specific retention times and the concentrations determined and expressed as mM concentrations per gram of sample for the raw fecal material and as mM concentrations per mL of fecal slurry.

16S rRNA sequencing

Extraction details:

For DNA extraction, the MoBioPowersoil 96 kit (now Qiagen Cat No./Id: 12955-4) was used with minor modifications. All samples were thawed on ice and 250ul of the 5x dilution slurry fecal (from ex vivo assay) from each sample were transferred to the Mobio High Throughput PowerSoil bead plate (12955-4 BP) for sample loading steps. Applicants then proceed the protocol as per manufacturer's protocol on the same day.

Library prep details, incl. primer:

Paired-end Illumina sequencing libraries were constructed using a two-step PCR approach targeting 16S rRNA genes V4 region, previously described by Preheim et al. (Preheim et al., 2013).

Sequencing details:

All paired-end libraries were multiplexed into lanes (at maximum 200 individual samples pooled per lane) and sequenced with paired end 150 bases on each end on a Illumina MiSeq platform.

Inulin-specific ELISA

Applicants use BioPAL's inulin immunoassay kit (BioPAL Worcester MA) to measure inulin concentration in filtrate from stool samples and follow manufacture's protocol. If stool samples came from *ex vivo* assay, they were already 5x dilution slurry faecal and needed to be centrifuged (10'000xg for 2 minutes) before passing the supernatant through a 0.2 $\mu$m syringe filter (Pall Corporation, Port Washington NY). If stool samples came from *in vivo* diet study, they were thawed on ice, homogenized to a 5x dilution slurry fecal (in PBS buffer) before being spinned down and filtered similarly to the ex vivo samples. The fecal filtrates are stored at -80°C, always thawed and kept on ice while taken out of the refrigerator, and diluted to an appropriate dilution to be in the detection range of the ELISA Inulin kit. For reference, the *ex vivo* inulin conditions need to be diluted at least 1'000 folds while the ex vivo control or in vivo samples only need to be diluted at least 10 folds.

Gut monolayer SCFA absorption measurements

Gut monolayer model:

Applicants used a gut monolayer system without the immune component, the Gut monolayer was prepared as previously described by WLK Chen et al. (Chen et al., 2017b) Briefly Caco-2 or C2BBe1 along with HT29-MTX-E21 cells (Sigma) were seeded onto rat tail collagen I-(corning 354236) coated Transwell inserts (Corning 3460) in a 9:1 ratio (1 x 105 cells/cm2) in 500 $\mu$l seeding medium. The top and bottom compartments of the Transwell Plate were fed with 500ul and 1.5ml of seeding medium. The medium was changed every 2-3 days. After 20 days of gut insert maturation, the cell monolayer was ready to use.

Cell monolayer integrity evaluation:

Barrier integrity was quantified by TEER (Trans-Epithelial Electrical Resistance) using the EVOM2 and the Enfohm-12 (World Precision Instruments) at 37°C. Applicants measured cell monolayer integrity at the beginning and the end of the 24h long experiment. At the in-between time points, Applicants surveilled the cell monolayer's (dis)continuity under the microscope.

SCFA sources:

In order to add SCFA to the mammalian cell media without significantly changing its pH, Applicants used the salt version of interest: Sodium Butyrate (Sigma, B5887), Sodium propionate (Alfa Aesar, A17440) and Sodium acetate (Sigma, S2889).

SCFA absorption experiment:

SCFAs are added to the gut cell media on the apical side while the basal side is unchanged, and the experiment is run for 24 hours. At each of the time points (0, 2, 4, 8 and 24h) Applicants collect 100$\mu$l from the apical side and 200$\mu$l from the basal side from each gut cell Transwell insert (previous experiments done by WLK Chen et al. have shown that the taken volume didn't affect the cell culture). Collected media from apical and basal side are stored at -80°C until SCFA analysis. Each condition is run in triplicates except for the controls. Applicants ran 5 different concentrations of SCFA combination. Butyrate:Propionate:Acetate ratio was kept constant across the different conditions, this ratio was 1:1:5 respectively. Different concentrations ran were 40mM, 20mM, 10mM, 5mM and 2mM of Butyrate with corresponding amounts of Propionate and Acetate. The controls were either single SCFA (5mM Butyrate, 5mM Propionate or 25mM Acetate) or without added SCFA.

At 24h, all the cells monolayers were washed, lysed and kept frozen at -80°C for further analysis if needed.

16S rRNA sequencing analysis

Raw paired-end 16 S rRNA Illumina sequencing reads were merged, demultiplexed, and quality trimmed with a cut-off of Q = 25 using usearch8, before being trimmed to a common length of 226 bases. Dereplicated reads were then clustered into OTUs to 97% identity using UPARSE (Edgar 2013). OTU centroids were assigned a taxonomy using the RDP classifier using an uncertainty cut-off of 0.5 (Cole 2005).

Machine learning

Random Forest Classifiers (RFCs) were built using the scikit-learn Python package. 5-fold cross-validation was used to construct an average Receiver Operator Characteristic (ROC) from which to compute an AUC.

**Example 1:**

*Ex vivo* measurements of SCFA production

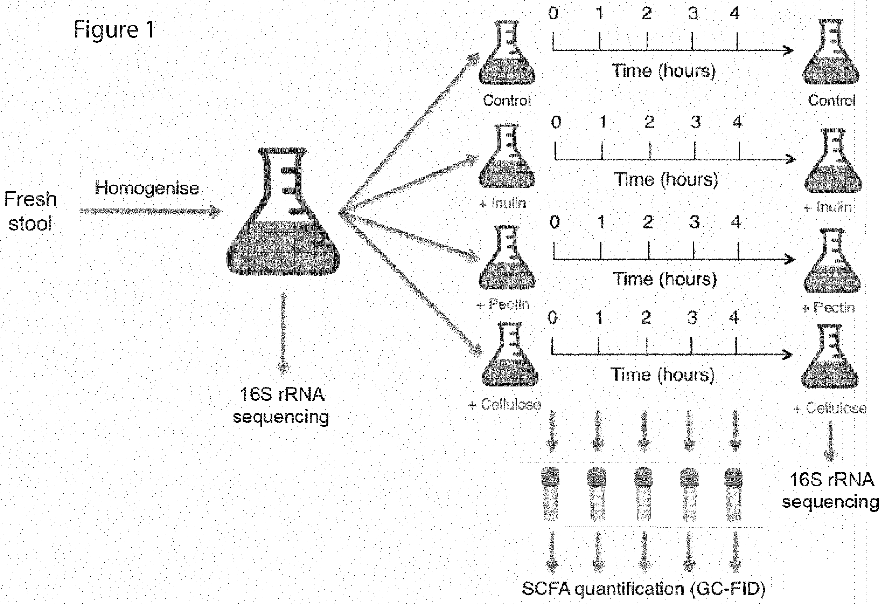

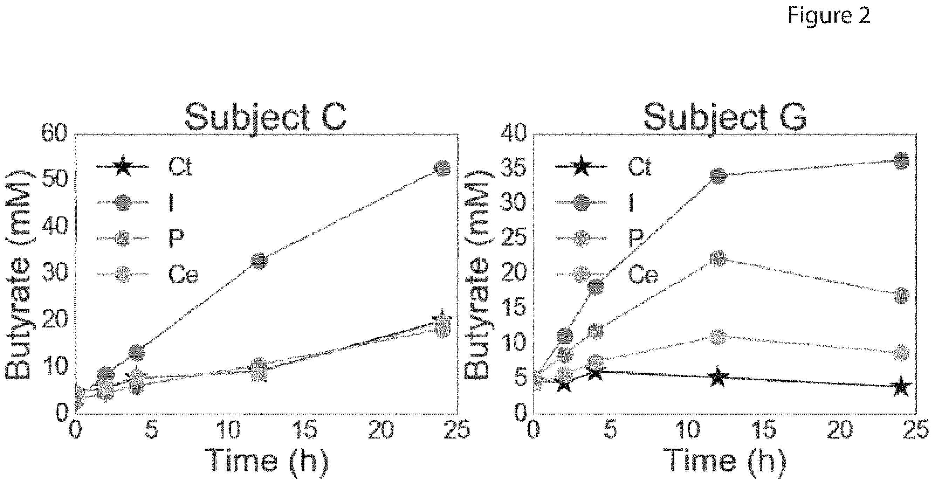

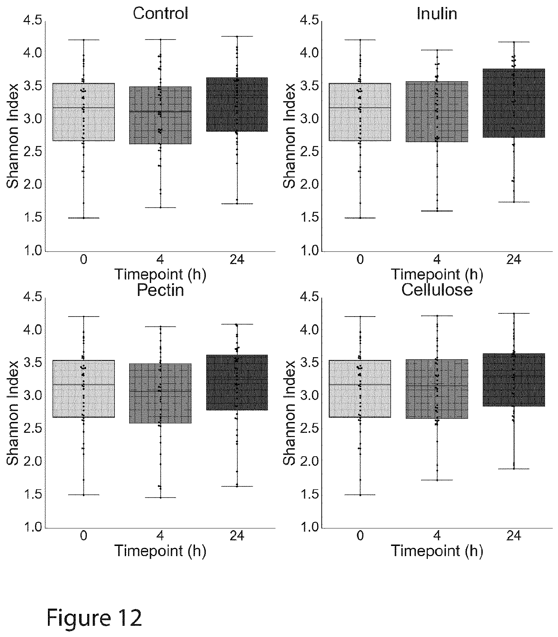

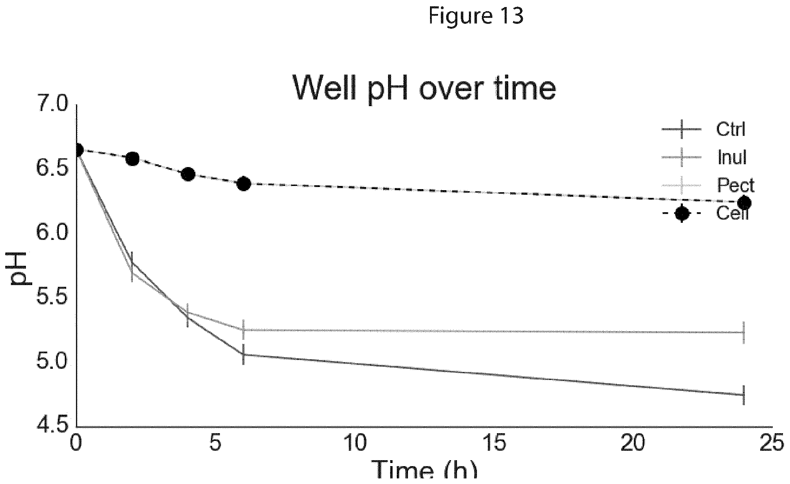

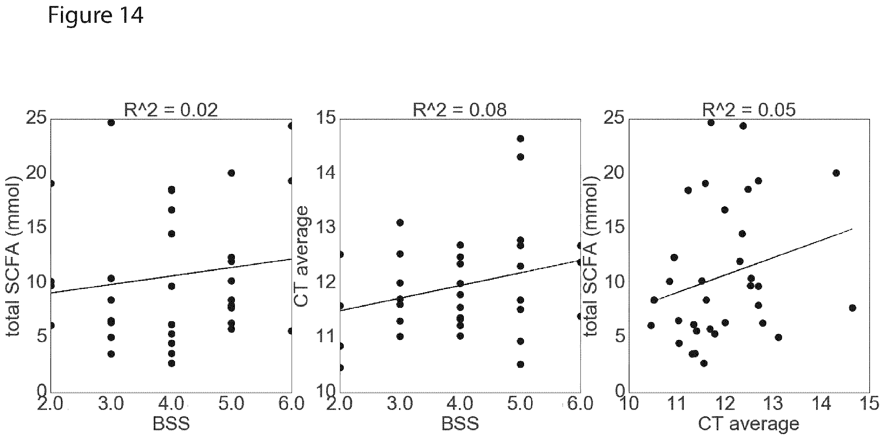

To measure SCFA production *ex vivo* in response to different dietary polysaccharides, stool from 40 healthy human participants was homogenized into a slurry in anaerobic conditions and spiked with inulin, pectin or cellulose (cf. Methods). The slurry was then allowed to evolve over time and samples obtained at regular intervals in order to quantify SCFA content at each timepoint (Fig. 1). In order to determine the appropriate sampling frequency, Applicants performed pilot experiments in which Applicants analysed the trajectory of each SCFA concentration over a 24h period. Applicants found that only a subset of participants appeared to converge to a final SCFA concentration prior to the 24h timepoint, but that all participants exhibited a linear production rate in the 0-4h time window (Fig.2). These data were in good agreement with concentrations of inulin measured from the stool over time using an inulin-specific ELISA assay: after 4h, a significant fraction of inulin substrate remained, but this was almost entirely consumed by 24h in five of the six participants tested (Fig. 11). Since Applicants sought to perform the experiments in an environment that best mimicked conditions in the colon, Applicants analysed 16S rRNA data and determined that community structure or diversity were not significantly altered between timepoints 0 and 4h, with minor changes being observed between 4h and 24h (Fig. 12). Moreover, changes in due to accumulation of acidic SCFAs was limited to a drop from approximately neutral to 5.5 during the first four hours (Fig. 13). Together, these data informed Applicants' decision to use the linear production regime observed between 0-4h as the most appropriate period in which to measure SCFA production *ex vivo* as a proxy for SCFA production *in vivo.* As a result, Applicants chose to measure two biological replicates for the timepoints 0, 2h and 4h, and compute production rates between timepoints 2h and 4h. Linear regressions between the sample's Bristol stool scale and total SCFA concentration produced, as well as between the CT values from the qPCR amplification of 16S rRNA from the stool did not suggest any apparent artefact induced by stool consistency or density, arguing that a correction factor for stool consistency or microbial load was not required and samples of various consistencies could be compared to one another (Fig. 14).

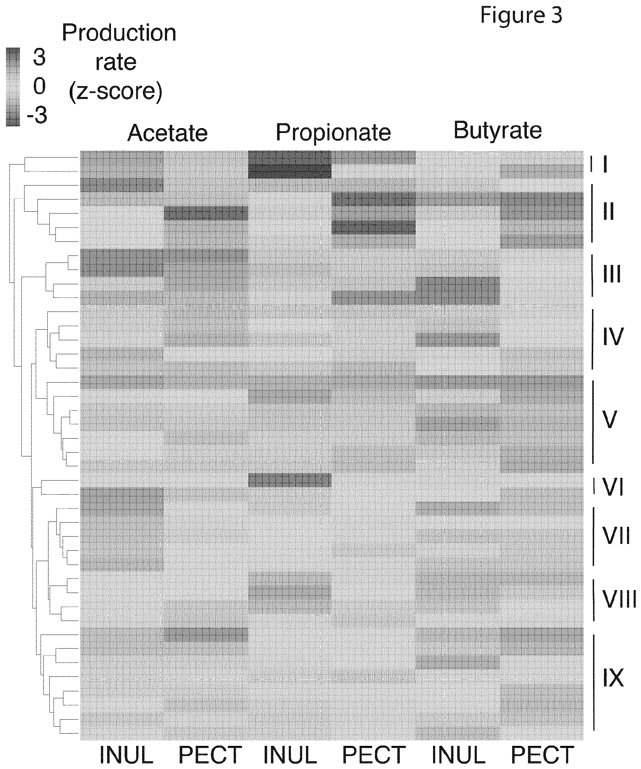

Applicants found that participants differed greatly in their SCFA production profiles (Fig.3). From the same dietary fiber input, the different microbiotas produced significantly different quantities and ratios of SCFAs. Applicants define the resulting SCFA production rates in response to different dietary fibers, quantified as standardized scores compared to the other participants in the dataset, as an individual's Microbial Metabolic Phenotype (MMP). Hierarchical clustering of MMPs indicated discernible groups: for example, individuals with MMP Type I were strong producers of propionate from inulin; in contrast, participants with MMP Type II were strong producers of propionate from pectin (Fig.3). Thus, improving an individual's production of a given SCFA will not necessarily rely on the same polysaccharide to reach the same effect; put differently, the same polysaccharide will have different effects in different individuals depending on their MMP. These data argue that there is significant heterogeneity in the healthy human population when it comes to functional degradation of fibers in the gut and the SCFAs produced, but that MMPs cluster into discernible types, which can be used to guide future dietary interventions.

**Example 2:**

Predicting microbial metabolic phenotype from community composition

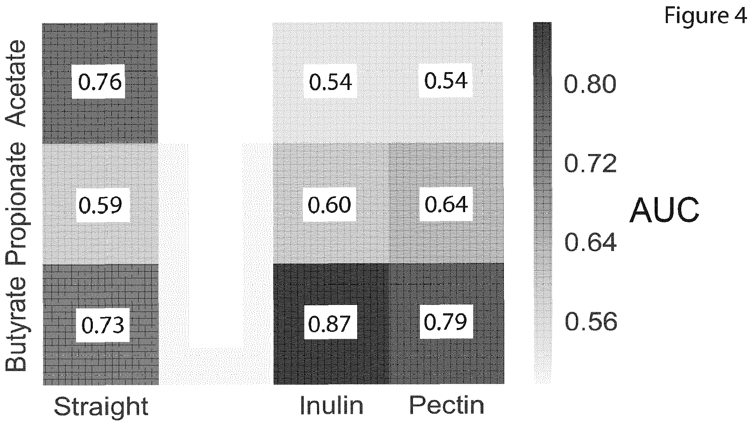

Applicants then asked the question whether Applicants could predict a participant's MMP from community composition alone, defined here as the relative abundances of 97% de novo OTUs obtained from 16S rRNA sequencing of the stool prior to incubation with the different fibers. Applicants trained Random Forest Classifiers (RFCs) to predict whether a given microbiota had a high or low production rate of a given SCFA in response to a given fiber, defined by a production rate z-score of greater than or equal to 0, or less than 0, respectively. Performance varied by SCFA, with the highest accuracies obtained in predicting butyrate production in response to inulin (AUC=0.87) and pectin (AUC=0.79) (Fig.4).

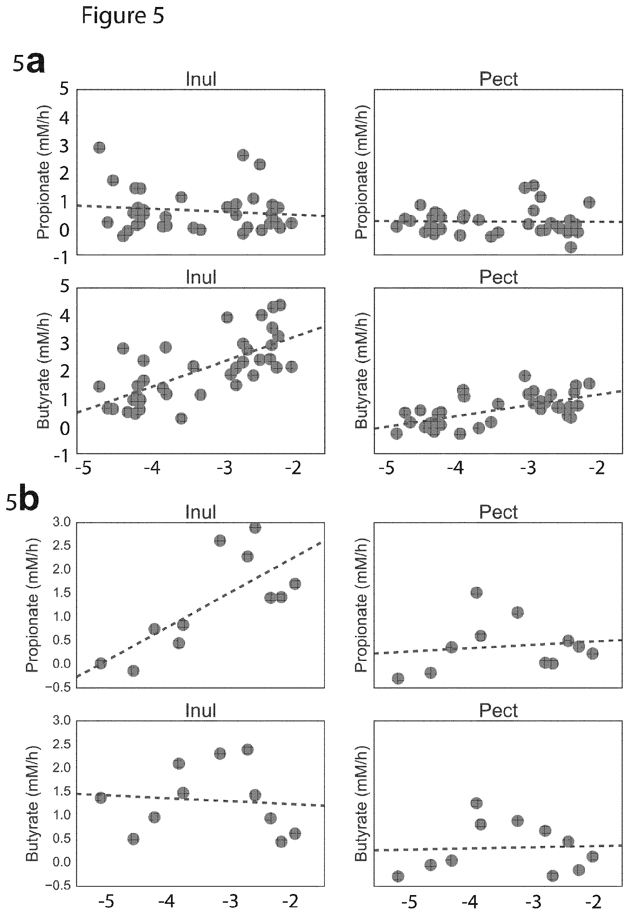

Applicants also tested whether straight stool SCFA contents could be predicted from 16S rRNA sequencing and found moderate predictive power for acetate and butyrate (AUC=0.76 and AUC=0.73, respectively). Though these data indicated that community composition is somewhat predictive of the resulting stool SCFA contents, manual inspection of specific OTU features that were highly ranked in terms of importance for the inulin- and pectin-specific RFCs found these too often only be associated with SCFA production in response to that specific fiber. For example, the relative abundance of an unassigned Lachnospiraceae OTU was only associated with butyrate production in response to inulin (Fig. 5a). In contrast, a specific Prevotella copri OTU only appeared to be associated with propionate production from inulin (Fig. 5b). These data are consistent with the fact that members of the Prevotella genus are known propionate producers (Chen et al., 2017a) and vary in their polysaccharide degradation capabilities (Filippis et al., 2019). Thus, a Type I MMP (Fig.3) is associated with a high relative abundance of the latter P. copri OTU. Similarly, known butyrate-producing families (e.g. Lachnospiraceae and Ruminococcaceae) are ranked highly in importance when RFCs for high/low SCFA production in response to inulin are trained on 16S rRNA data collapsed at the family level. These results show that individual OTUs are predictive of SCFA production capability from specific polysaccharides, likely due to their specific polysaccharide degradation machinery and internal fermentation pathways.

**Example 3:**

Stability of an individual's MMP through time

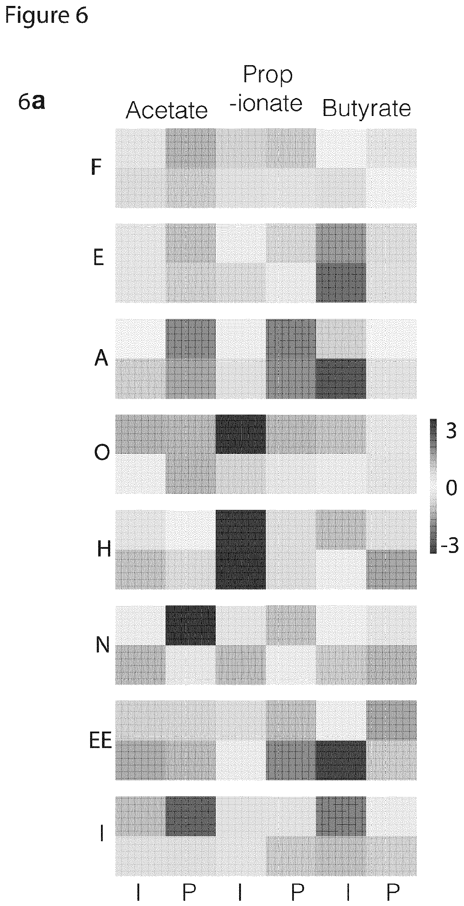

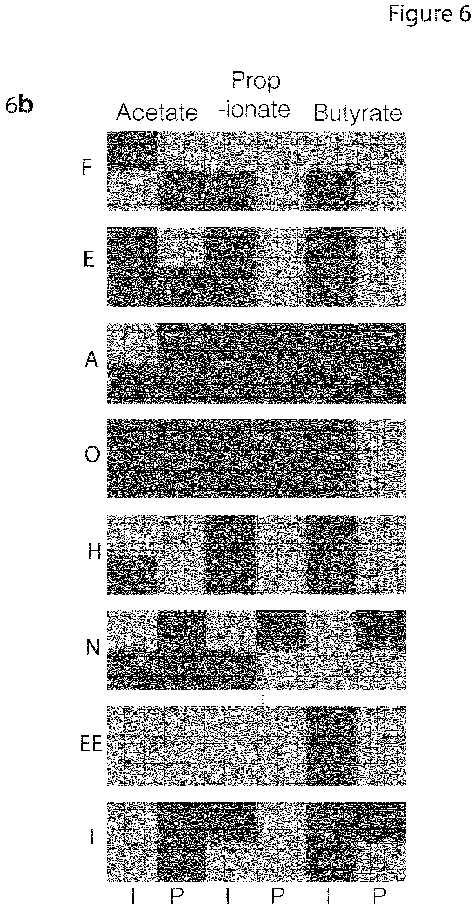

While it is known that the gut microbiota of individuals can be relatively stable for long periods of time in the absence of large perturbations, it is unclear whether an individual's MMP will also be similarly stable through time. Applicants therefore repeated the experiment for eight participants at timepoints separated by at least 6 months (Fig. 6a). Though some variability between timepoints was observed, the extrema of each individual's MMP were generally preserved (Fig. 6b). A Fisher test on the contingency table resulting from pairwise comparison of each SCF A: fiber pair at the two timepoints for all individuals indicated that this stability was statistically significant (p=0.003; two-tailed Fisher test). These results were consistent with the fact that MMPs are associated with the relative abundance of specific members of the microbiota: an individual with a high relative abundance of the aforementioned P. copri OTU is likely to retain a high relative abundance of this OTU during the six month period between timepoints compared to the general population, thus retaining their ability to produce high levels of propionate from inulin through time.

**Example 4:**

Model of SCFA production

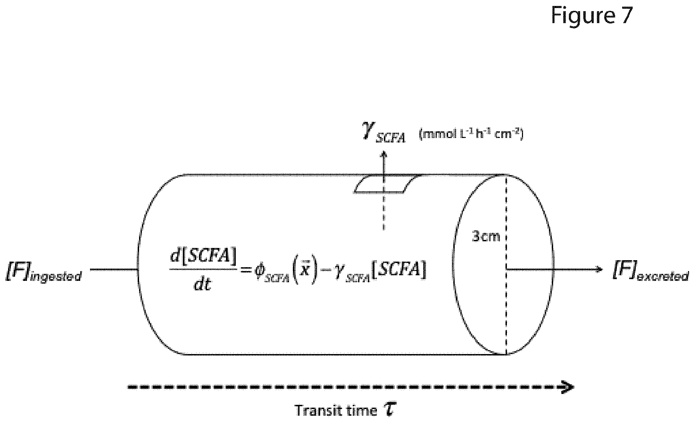

In order to better understand microbial SCFA production in vivo, Applicants sought to develop a quantitative model of the process (Fig. 7). In a given participant, when a quantity of dietary fiber, [F], is consumed, it is fermented into acetate, propionate and butyrate with rates that are functions of their specific microbiota. As defined, a person's MMP is the aggregate total response of an individual's fecal microbiota to a challenge with specific dietary fibers. The process of producing SCFAs from fiber inputs requires several steps (Fig. 15b). The first step involves breaking the fiber/polysaccharide (F) into smaller oligo- and monosaccharides (O), usually through a hydrolysis reaction encoded by extracellular enzymes (Sonnenburg et al., 2010); the second step consists of fermenting O into a reduced biochemical species P (e.g. lactate, acetate, pyruvate), which can act as a substrate for the third step, the final fermentation reaction that leads to the final product or SCFA in question. A person's MMP is thus the bulk total of all these individual reactions (Fig. 15a).

In principle, some or all of the dietary fiber can be excreted in the stool without having undergone any fermentation in the colon. Thus, Applicants sought to determine whether a consumed quantity of dietary fiber can be expected to be completely fermented in the time taken for a typical bolus of food to transit through the gut. Applicants measured fecal inulin concentrations from a previous in vivo study where participants were given 10g of inulin from the same vendor used in our ex vivo experiments as a daily supplement against a constant dietary background (Gurry et al., 2018), and found that fecal inulin concentrations ranged from undetectable to 25 mg/L on the days following inulin consumption (Fig. 8), compared to an input concentration of approximately 10g/L (if we estimate the volume of stool in the gut to be on the order of 1L; cf. Methods). These data suggest that a bolus of inulin is almost entirely degraded by the microbiota upon passage of the stool, consistent with Applicants' measurements of inulin depletion over 24h ex vivo (Fig. 11). Applicants therefore assume this excretion rate of the dietary fiber is zero and that it is almost entirely consumed within the gut (i.e. [F]excreted = 0).

Gut luminal concentrations of microbially-derived SCFAs is a balance between net production in the lumen and absorption by the host epithelium. Unfortunately, quantitative understanding of the rate of absorption of a given SCFA by cells in the gut epithelia is limited. Previous data collected using a dialysis bag technique suggest that SCFA absorption rate is linear with concentration at typical physiological concentrations of SCFAs (McNeil et al., 1978). Moreover, studies have shown that SCFA absorption exhibits an unexpectedly modest pH-dependence (Bugaut, 1987). Applicants therefore modelled the absorption rate as proportional to the luminal SCFA concentration. Applicants sought to estimate the order of magnitude of this rate constant, to better understand the fate of SCFAs produced by the microbiota in the colon (i.e. the balance between quantities absorbed versus quantities excreted). For this purpose, Applicants used CaCo cell monolayers grown in a trans-well and measured SCFA concentrations from the media sampled from the apical and basal sides of the monolayer over a duration of 24h (cf. Methods and Supporting Information for details). Assuming the SCFA production rates measured *ex vivo* are representative of *in vivo* rates of production with the same inputs, and explicitly including the absorption rate parameters, Applicants obtain the following system of phenomenological equations describing the time evolution of each SCFA's concentration in the colonic lumen:

$$\frac{d[A]}{dt}=\phi_A\left(\vec{x}\right)-\gamma_A[A]$$

$$\frac{d[P]}{dt}=\phi_P\left(\vec{x}\right)-\gamma_P[P]$$

$$\frac{d[B]}{dt}=\phi_B\left(\vec{x}\right)-\gamma_B[B]$$

where $\phi_A(\vec{x})$, $\phi_P(\vec{x})$ and $\phi_B(\vec{x})$ are bulk production rates of acetate, propionate and butyrate respectively, produced in response to a given input concentration of fiber [F], defining an individual's MMP.

In order to better understand the impact of the absorption rate constant on quantities of SCFA absorbed by the gut epithelium, Applicants used this model to calculate the amount of butyrate absorbed in a given participant as a function of this rate constant:

$$B_{absorbed}\left(\tau\right)=V_{colon}S_{colon}\int_0^\tau \gamma_B[B](t)dt,$$

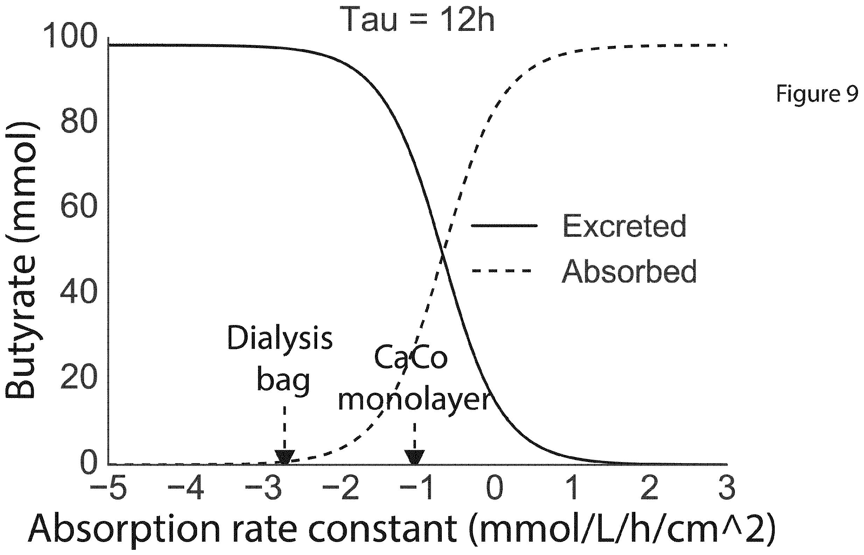

where $B_{absorbed}$ is the quantity (in mmol) of butyrate absorbed, $V_{colon}$ and $S_{colon}$ are the volume and surface areas of the colon, respectively, [B] is the concentration of butyrate in the colon, and $\gamma_B$ is the absorption rate of butyrate in mmol L-1h-1cm-2. Applicants found that, assuming a transit time of 12 hours, the rate constants Applicants use in this study (dialysis bag and CaCo monolayer-derived rate constants) have significantly different effects on the resulting dynamics: in the case of the dialysis bag constant (0.0019mmol L-1h-1cm-2), the majority of produced butyrate is excreted, while in the case of CaCo monolayer (0.091mmol L-1h-1cm-2), a significant quantity is absorbed (~40%) while the remainder is excreted (Fig. 9). This indicates that, depending on the value of the absorption rate constant, the relationship between excreted stool SCFA quantities and quantities of SCFA produced and absorbed in the gut are not necessarily related.

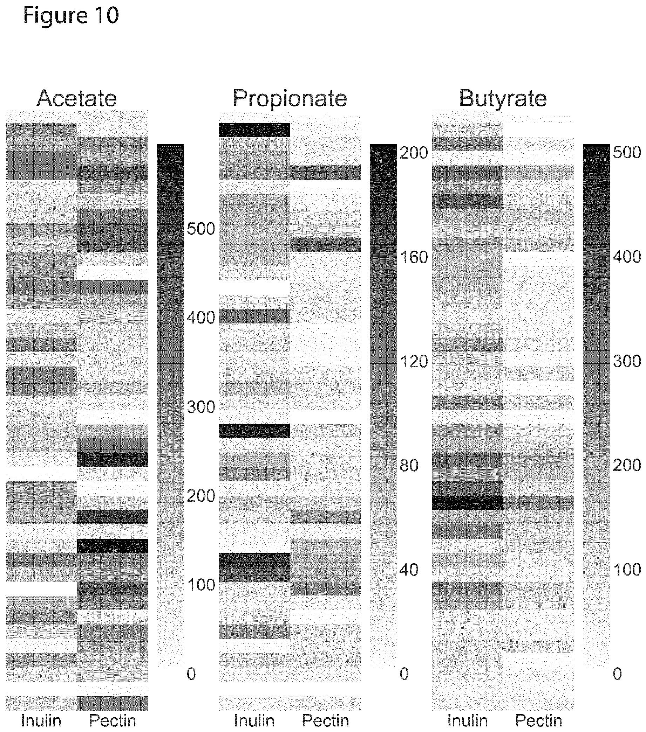

Transit time is a significant variable controlling the quantities of SCFA absorbed by the gut in the orders of magnitude of the absorption rates considered. This relationship holds across different participants with different production rates of butyrate from the same input, related by a scaling factor. However, Applicants' model does not account for depletion of the fiber substrate, which measurements of stool concentrations of inulin (Fig. 8 and Fig. 11) suggest is likely to occur by the time the stool is excreted. Of course, slowing colonic transit time is therefore only an effective way of increasing absorbed SCFAs for as long as there remain SCFAs to absorb and fiber substrate to ferment. Nonetheless, it is clear from Applicants' model that, despite assuming equal absorption rate constants across participants and a transit time of 12h, different individuals absorb different quantities of an SCFA from a given fiber (Fig. 10), mirroring their differences in production and overall MMP Production always higher than absorption and absorption is linearly related to produced concentration. Changing absorption rate constant parameters only affects the scale of absorbed quantities but the relative ratios between individuals are unchanged.

**Example 5:**

Model of microbial SCFA production

Figure 15a illustrates the steps in the degradation of dietary fibers into SCFAs that involve the microbiota. In this framework, distinct equations describing the time-evolution of the concentration of each chemical species exist for each type of monomer M, intermediate P, and SCFA. In reality, the system is sparse, as the rate constants $\pi_{i1}$, $\pi_{j2}$, and $\pi_{k3}$ are zero or close to zero in the vast majority of OTUs due to the fact that only a small subset of OTUs have the necessary biochemical capabilities. For a given concentration of dietary fiber [F], the combination of all contributing equations to a given rate of

$$\frac{d[SCFA]}{dt}$$

production of a specific SCFA can be summarized by a single parameter, $\phi_{SCFA}(\vec{x})$, which is a function of the composition of the microbiota (Fig. 15b). Thus, in *ex vivo* conditions,

$$\frac{d[SCFA]}{dt} \equiv \phi_{SCFA}\left(\vec{x}\right).$$

**Example 6**:

Estimation of absorbed SCFAs across the colon's epithelium

Applicants approximate the colon as a cylinder 3cm in diameter. This corresponds to a circumference of approximately 9.42cm. Thus, each 1cm-thick cross-sectional segment of the colon has a surface area of approximately 9.42cm2, which Applicants round up to 10cm2. Estimating the length of the colon at 150cm, Applicants reach a total surface area of approximately 1,500cm2. Assuming the colon is filled with stool, Applicants reach a total internal stool volume of 1,060cm3, which Applicants approximate as 1L.

The absorption rate constant of a given SCFA estimated using the CaCo cell monolayer experiments, YSCFA, is in units of mol cm-2 h-1L-1. Thus, the absorption rate of the entire colon is 1,500ϒSCFA mol h-1L-1 of each SCFA. Based on Applicants' approximation of 1L of total stool, this amounts to an absorption rate of approximately 1,500ϒSCFA mol h-1 for each SCFA.

**Example 7:**

Comparison between *ex vivo* and *in vivo*

Applicants next sought to determine whether changes in SCFAs or community structure observed under *ex vivo* conditions were in agreement with what could theoretically be measured *in vivo.* For this purpose, Applicants turned to a dataset from a previous study in which participants were placed on a fixed diet consisting entirely of a fiber-impoverished, liquid, nutritional meal supplement for a period of six days (Gurry et al., 2018). In the latter three days, participants were randomized to a spike-in, to be consumed at a prescribed dose daily against the constant liquid diet background. These spike-ins included inulin, pectin, and cellulose, and used the exact same sources of these three fibers, providing us with an ideal comparison dataset.

Under *ex vivo* conditions, Applicants observed complete degradation of the inulin bolus in certain participants (Fig. 6a). Applicants sought to determine whether a similar extent of degradation could be observed *in vivo* in participants consuming an inulin bolus at similar concentrations. Residual inulin was therefore quantified from the stool using an inulin-specific ELISA assay. Applicants find that, as expected, no detectable inulin can be found in the stool of participants on days 1, 2 and 3, but that inulin is detectable on days after which inulin was consumed in certain stool samples (Fig. 8). However, the inulin detected on these days accounts for a tiny minority of the total inulin consumed (10g/day), which assuming a total daily stool volume of 1L, equates to approximately 10g/L, the concentration used in the ex vivo experiments. This suggests that the majority of accessible inulin is also consumed in vivo in the typical stool passage time following ingestion. Moreover, since 10g is a significantly larger dose of inulin than would ordinarily be consumed in a typical diet, Applicants can conclude that the majority of inulin consumed in an ordinary diet in a form similarly accessible to the inulin powder used for these experiments is fermented *in vivo*. Of course, it is likely that inulin from natural sources and typical dietary fiber sources do not contain the fiber in as accessible form as the purified form used in these experiments.

## References

Bugaut, M. (1987). Occurrence, absorption and metabolism of short chain fatty acids in the digestive tract of mammals. Comp. Biochem. Physiol. Part B Comp. Biochem. 86, 439-472. Chen, T., Long, W., Zhang, C., Liu, S., Zhao, L., and Hamaker, B.R. (2017a). Fiber-utilizing capacity varies in Prevotella - versus Bacteroides -dominated gut microbiota. Sci. Rep. 7, 2594.

Chen, W.L.K., Edington, C., Suter, E., Yu, J., Velazquez, J.J., Velazquez, J.G., Shockley, M., Large, E.M., Venkataramanan, R., Hughes, D.J., et al. (2017b). Integrated gut/liver microphysiological systems elucidates inflammatory inter-tissue crosstalk. Biotechnol. Bioeng. 114, 2648-2659.

Cole, J. R. et al. (2005) The Ribosomal Database Project (RDP-II): sequences and tools for high-throughput rRNA analysis. Nucleic Acids Res. 33, D294-D296 (2005).

Davie, J.R. (2003). Inhibition of Histone Deacetylase Activity by Butyrate. J. Nutr. 133, 2485S-2493S.

De Vadder, F., Kovatcheva-Datchary, P., Goncalves, D., Vinera, J., Zitoun, C., Duchampt, A., Bäckhed, F., and Mithieux, G. (2014). Microbiota-Generated Metabolites Promote Metabolic Benefits via Gut-Brain Neural Circuits. Cell 156, 84-96.

Duncan, S.H., Barcenilla, A., Stewart, C.S., Pryde, S.E., and Flint, H.J. (2002). Acetate Utilization and Butyryl Coenzyme A (CoA):Acetate-CoA Transferase in Butyrate-Producing Bacteria from the Human Large Intestine. Appl. Environ. Microbiol. 68, 5186-5190.

Edgar, R. C. (2013) UPARSE: highly accurate OTU sequences from microbial amplicon reads. Nat. Methods 10, 996-998.

Filippis, F.D., Pasolli, E., Tett, A., Tarallo, S., Naccarati, A., Angelis, M.D., Neviani, E., Cocolin, L., Gobbetti, M., Segata, N., et al. (2019). Distinct Genetic and Functional Traits of Human Intestinal Prevotella copri Strains Are Associated with Different Habitual Diets. Cell Host Microbe 25, 444-453.e3.

Gurry, T., Gibbons, S.M., Nguyen, L.T.T., Kearney, S.M., Ananthakrishnan, A., Jiang, X., Duvallet, C., Kassam, Z., and Alm, E.J. (2018). Predictability and persistence of prebiotic dietary supplementation in a healthy human cohort. Sci. Rep. 8, 12699.

Kuo, S.-M. (2013). The Interplay Between Fiber and the Intestinal Microbiome in the Inflammatory Response. Adv. Nutr. Int. Rev. J. 4, 16-28.

Machiels, K., Joossens, M., Sabino, J., Preter, V.D., Arijs, I., Eeckhaut, V., Ballet, V., Claes, K., Immerseel, F.V., Verbeke, K., et al. (2013). A decrease of the butyrate-producing species Roseburia hominis and Faecalibacterium prausnitzii defines dysbiosis in patients with ulcerative colitis. Gut gutjnl-2013-304833.

McNeil, N.I., Cummings, J.H., and James, W.P. (1978). Short chain fatty acid absorption by the human large intestine. Gut 19, 819-822.

Perry, R.J., Peng, L., Barry, N.A., Cline, G.W., Zhang, D., Cardone, R.L., Petersen, K.F., Kibbey, R.G., Goodman, A.L., and Shulman, G.I. (2016). Acetate mediates a microbiome-brain-$\beta$-cell axis to promote metabolic syndrome. Nature 534, 213-217.

Preheim, S.P., Perrotta, A.R., Martin-Platero, A.M., Gupta, A., and Alm, E.J. (2013). Distribution-Based Clustering: Using Ecology to Refine the Operational Taxonomic Unit. Appl Env. Microbiol 79, 6593-6603.

Roediger, W.E. (1980). Role of anaerobic bacteria in the metabolic welfare of the colonic mucosa in man. Gut 21, 793-798.

Sanna, S., Zuydam, N.R. van, Mahajan, A., Kurilshikov, A., Vila, A.V., Võsa, U., Mujagic, Z., Masclee, A.A.M., Jonkers, D.M.A.E., Oosting, M., et al. (2019). Causal relationships among the gut microbiome, short-chain fatty acids and metabolic diseases. Nat. Genet. 1.

Sonnenburg, E.D., Zheng, H., Joglekar, P., Higginbottom, S.K., Firbank, S.J., Bolam, D.N., and Sonnenburg, J.L. (2010). Specificity of Polysaccharide Use in Intestinal Bacteroides Species Determines Diet-Induced Microbiota Alterations. Cell 141, 1241-1252.

Zhao, L., Zhang, F., Ding, X., Wu, G., Lam, Y.Y., Wang, X., Fu, H., Xue, X., Lu, C., Ma, J., et al. (2018). Gut bacteria selectively promoted by dietary fibers alleviate type 2 diabetes. Science 359, 1151-1156.

Zimmerman, M.A., Singh, N., Martin, P.M., Thangaraju, M., Ganapathy, V., Waller, J.L., Shi, H., Robertson, K.D., Munn, D.H., and Liu, K. (2012). Butyrate suppresses colonic inflammation through HDAC1-dependent Fas upregulation and Fas-mediated apoptosis of T cells. Am. J. Physiol. - Gastrointest. Liver Physiol. 302, G1405-G1415.

## Claims

1. A method for predicting a response to different dietary fibres from nucleic acid sequences, said response to different dietary fibres being defined based on the fermentation or metabolic capabilities of a subject's gut microbiota assessed by its short chain fatty acid (SCFA) production rate in response to said different dietary fibres, the method being applied to abundance data of specific microbiota-derived nucleic acids obtained from prior collected stabilized biological sample of said subject, the method comprising the steps of:

   - isolating and extracting said specific microbiota-derived nucleic acids from said stabilized biological sample, wherein the specific sequences of said specific microbiota-derived nucleic acids are identified from a training database containing metagenomics sequencing data of the microbiota from healthy subjects, or subjects suffering from a particular disease, paired with *in vitro* measured data pertaining to the production rates of SCFAs and other fermentation metabolites from different dietary fibers in such subjects, said SCFA and other fermentation metabolites being selected from the list comprising propionate, butyrate, acetate, lactate, formate, succinate, iso-butyrate, valerate and iso-valerate;
   - determining and quantifying the relative abundance of said specific microbiota-derived nucleic acids from said stabilized biological sample to generate a first input, wherein said specific microbiota-derived nucleic acids consist in gene sequences specific to glycoside and/or polysaccharide hydrolases capable of hydrolyzing or cleaving complex polysaccharides of said different dietary fibres, as well as gene sequences specific to key enzymes from bacterial acetate, propionate and butyrate production pathways and/or other enzymes responsible for the degradation of said different dietary fibers;
   - applying a machine learning algorithm trained on the training database to predict from these specific microbiota-derived nucleic acid sequences the production rate of SCFAs and other fermentation metabolites in response to challenge with different dietary fibers so as to obtain a response to said different dietary fibres.

2. The method according to claim 1, **characterized in that** the training database comprises data classified according to a predetermined set of classification groups, wherein the assessment of said fermentation or metabolic capabilities of a subject's gut microbiota comprises classifying the subject according to a specific type or phenotype.

3. The method according to any one of claims 1-2, **characterized in that** the machine learning algorithm further comprises a set of algorithms for linking the data associated with the subject's microbiome to a certain medical condition, physical condition, or likely responsiveness to a certain therapy.

4. The method according to any one of claims 1-3, further comprising steps for stratifying the subject to different treatment plans, including personalized dietary recommendations or other therapeutic strategies aiming to improve the subject's microbiota's fermentation capabilities so as to build the subject's microbiome profile.

5. The method of claim 4, wherein said personalized dietary recommendations are formulated by identifying the dietary fibers inputs that result in the highest and lowest production of metabolites of interest by the subject's microbiota so as to establish personal care product to the subject.

6. The method of claim 4, wherein said therapeutic strategies comprises one or more prebiotics, one or more probiotics, one or more antibiotics, or any other drug for therapeutic treatment disease or disorder with a metabolic and/or immunological and/or inflammatory involvement.

7. The method of claim 4, wherein said different treatment plans include predisposition to a disease or disorder with a metabolic and/or immunological and/or inflammatory involvement which is selected from the group comprising obesity, metabolic syndrome or diseases, diabetes mellitus such as Type-2 diabetes, insulin-deficiency related disorders, insulin-resistance related disorders, intestinal gluconeogenesis disorders, inflammation disorders, inflammatory Bowel disease, systemic or local inflammation in the context of neurodegeneration, rheumatoid arthritis, depression or anxiety disorders, food intolerance, diarrhea, constipation, colitis and enteritis, allergies and cancer immunotherapy applications.

8. The method according to any one of the preceding claims, wherein the subject's biological sample is selected from the group comprising: a rectal swab, a fecal sample, a biopsy or a mucosal layer sample.

9. The method according to any one of the preceding claims, wherein the determination and quantification of the relative abundance of said specific microbiota-derived nucleic acids is performed by either DNA sequencing specific marker genes including 16S rRNA, shotgun metagenomics DNA sequencing, PCR or qPCR of specific genes, or any method for nucleic acid quantification including capillary electrophoresis.

10. The method according to any one of the preceding claims, wherein said machine-learning algorithm comprises a supervised learning procedure and optionally at least one procedure selected from the group consisting of clustering, support vector machine, linear modeling, k-nearest neighbors analysis, decision tree learning, ensemble learning procedure, neural networks, probabilistic model, graphical model, Bayesian network, and association rule learning.

11. The method according to any one of the preceding claims, wherein said different dietary fibres are complex polysaccharides or other dietary ingredients selected from the group comprising: inulins, fructo-oligosaccharide, resistant starch, lignin, tannin, cellulose, hemicelluloses (including xyloglucans, glucuronoxylans, arabinoglucuronoxylans, arabinoxylans, heteroxylans, mannans, and beta-glucans), psyllium, polydextrose, chitin, chitosan, pectins (including homogalacturonans/polygalacturonans, and rhamnogalacturonans I and II), arabinan, and konnyaku / konjac (glucomannan).

12. The method according to any one of the preceding claims, wherein the other enzymes responsible for the degradation of said different dietary fibres are selected from the group comprising bacterial, archaeal, or fungal originated from the subject's gut microbiota.

13. A computer software product, comprising a computer-readable medium in which program instructions are stored, which instructions, when read by a data processor, cause the data processor to receive data collected from a subject's sample, and to execute the method according to any of claims 1-12.

14. An apparatus for predicting a response of a subject to different dietary fibres, the apparatus comprising: a user interface configured to receive a subject's sample; a module for extracting specific microbiota-derived nucleic acids from said subject's sample and for determining and quantifying the relative abundance of said specific microbiota-derived nucleic acids; and a data processor having a computer-readable medium storing the computer software product of claim 13.

15. The apparatus according to claim 14, further comprising a PCR kit for sequencing and quantifying the relative abundance of said specific microbiota-derived nucleic acids identified from a training database, said PCR kit comprising

   a. primers specific for nucleic acid sequences that are identified from the training database as most predictive of fiber response; and
   b. optionally, primers specific for sequences from the genes and metabolic pathways specific to key enzymes from bacterial acetate, propionate and butyrate production pathways, as well as other enzymes responsible for the degradation of said different dietary fibres.

**Patentansprüche**

1. Verfahren zur Vorhersage einer Reaktion auf verschiedene Ballaststoffe aus Nukleinsäuresequenzen, wobei die Reaktion auf verschiedene Ballaststoffe basierend auf den Fermentations- oder Stoffwechselfähigkeiten der Darmmikrobiota eines Probanden definiert wird, die anhand ihrer Produktionsrate kurzkettiger Fettsäuren (SCFA) in Reaktion auf die verschiedenen Ballaststoffe beurteilt werden, und das Verfahren auf Häufigkeitsdaten spezifischer, aus der Mikrobiota stammender Nukleinsäuren angewendet wird, die aus einer zuvor gesammelten stabilisierten biologischen Probe des Probanden erhalten wurden, wobei das Verfahren die folgenden Schritte umfasst:

   - Isolieren und Extrahieren der spezifischen, aus der Mikrobiota stammenden Nukleinsäuren aus der stabilisierten biologischen Probe, wobei die spezifischen Sequenzen der spezifischen, aus der Mikrobiota stammenden Nukleinsäuren aus einer Trainingsdatenbank identifiziert werden, die Metagenomik-Sequenzierungsdaten der Mikrobiota von gesunden Probanden oder von Probanden, die an einer bestimmten Krankheit leiden, gepaart mit *in vitro* gemessenen Daten bezüglich der Produktionsraten von SCFAs und anderen Fermentationsmetaboliten aus verschiedenen Ballaststoffen in diesen Probanden enthält, wobei die SCFA und die anderen Fermentationsmetaboliten aus der Liste ausgewählt werden, die Propionat, Butyrat, Acetat, Lactat, Formiat, Succinat, Isobutyrat, Valerat und Isovalerat umfasst;
   - Bestimmen und Quantifizieren der relativen Häufigkeit der spezifischen, aus der Mikrobiota stammenden Nukleinsäuren aus der stabilisierten biologischen Probe, um einen ersten Input zu erzeugen, wobei die spezifischen, aus der Mikrobiota stammenden Nukleinsäuren aus Gensequenzen, die spezifisch für Glykosid- und/oder Polysaccharidhydrolasen sind, die in der Lage sind, komplexe Polysaccharide der verschiedenen Ballaststoffe zu hydrolysieren oder zu spalten, sowie Gensequenzen bestehen, die spezifisch für Schlüsselenzyme aus bakteriellen Acetat-, Propionat- und Butyrat-Produktionswegen und/oder Enzyme sind, die für den Abbau der verschiedenen Ballaststoffe verantwortlich sind;

- Anwenden eines anhand der Trainingsdatenbank trainierten Algorithmus für maschinelles Lernen, um aus diesen spezifischen, aus der Mikrobiota stammenden Nukleinsäuresequenzen die Produktionsrate von SCFAs und anderen Fermentationsmetaboliten in Reaktion auf die Provokation mit verschiedenen Ballaststoffen vorherzusagen, um eine Reaktion auf die verschiedenen Ballaststoffe zu erhalten.

2. Verfahren Anspruch 1, **dadurch gekennzeichnet, dass** die Trainingsdatenbank Daten umfasst, die gemäß einem vorbestimmten Satz von Klassifizierungsgruppen klassifiziert sind, wobei die Beurteilung der Fermentations- oder Stoffwechselfähigkeiten der Darmmikrobiota eines Probanden Klassifizieren des Probanden gemäß einem spezifischen Typ oder Phänotyp umfasst.

3. Verfahren nach einem der Ansprüche 1-2, **dadurch gekennzeichnet, dass** der Algorithmus für maschinelles Lernen ferner einen Satz von Algorithmen zum Verknüpfen der mit dem Mikrobiom des Probanden assoziierten Daten mit einem bestimmten medizinischen Zustand, einem bestimmten körperlichen Zustand oder einer wahrscheinlichen Reaktion auf eine bestimmte Therapie umfasst.

4. Verfahren nach einem der Ansprüche 1-3, ferner umfassend Schritte zum Einteilen des Probanden in verschiedene Behandlungspläne, einschließlich personalisierter Ernährungsempfehlungen oder anderer therapeutischer Strategien, die darauf abzielen, die Fermentationsfähigkeiten der Mikrobiota des Probanden zu verbessern, um das Mikrobiomprofil des Probanden zu erstellen.

5. Verfahren nach Anspruch 4, wobei die personalisierten Ernährungsempfehlungen formuliert werden, indem die Ballaststoffzufuhren identifiziert werden, die zur höchsten und niedrigsten Produktion von Metaboliten von Interesse durch die Mikrobiota des Probanden führen, um ein Körperpflegeprodukt für den Probanden zu etablieren.

6. Verfahren nach Anspruch 4, wobei die therapeutischen Strategien ein oder mehrere Präbiotika, ein oder mehrere Probiotika, ein oder mehrere Antibiotika oder ein anderes Arzneimittel zur therapeutischen Behandlung von Krankheiten oder Störungen mit metabolischer und/oder immunologischer und/oder entzündlicher Beteiligung umfassen.

7. Verfahren nach Anspruch 4, wobei die verschiedenen Behandlungspläne eine Prädisposition für eine Krankheit oder Störung mit metabolischer und/oder immunologischer und/oder entzündlicher Beteiligung umfassen, die aus der Gruppe ausgewählt wird, die Fettleibigkeit, metabolisches Syndrom oder metabolische Krankheiten, Diabetes mellitus wie Typ-2-Diabetes, mit Insulinmangel verbundene Störungen, mit Insulinresistenz verbundene Störungen, Störungen der intestinalen Gluconeogenese, entzündliche Störungen, entzündliche Darmerkrankungen, systemische oder lokale Entzündungen im Zusammenhang mit Neurodegeneration, rheumatoide Arthritis, Depressionen oder Angststörungen, Nahrungsmittelunverträglichkeit, Durchfall, Verstopfung, Colitis und Enteritis, Allergien und Anwendungen in der Krebsimmuntherapie umfasst.

8. Verfahren nach einem der vorhergehenden Ansprüche, wobei die biologische Probe des Probanden aus der Gruppe ausgewählt wird, die Folgendes umfasst: einen Rektalabstrich, eine Stuhlprobe, eine Biopsie oder eine Schleimhautprobe.

9. Verfahren nach einem der vorhergehenden Ansprüche, wobei die Bestimmung und Quantifizierung der relativen Häufigkeit der spezifischen, aus der Mikrobiota stammenden Nukleinsäuren entweder durch DNA-Sequenzierung spezifischer Markergene, einschließlich 16S rRNA, Shotgun-Metagenomik-DNA-Sequenzierung, PCR oder qPCR spezifischer Gene oder ein beliebiges Verfahren zur Nukleinsäurequantifizierung, einschließlich Kapillarelektrophorese, erfolgt.

10. Verfahren nach einem der vorhergehenden Ansprüche, wobei der Algorithmus für maschinelles Lernen eine Prozedur überwachten Lernens und optional mindestens eine Prozedur umfasst, die aus der Gruppe bestehend aus Clustering, Support Vector Machine, linearer Modellierung, K-Nearest-Neighbors-Analyse, Entscheidungsbaum-Lernen, Ensemble-Lernprozedur, neuronalen Netzwerken, probabilistischem Modell, grafischem Modell, Bayes-Netzwerk und Assoziationsregel-Lernen ausgewählt wird.

11. Verfahren nach einem der vorhergehenden Ansprüche, wobei die verschiedenen Ballaststoffe komplexe Polysaccharide oder andere Nahrungsbestandteile sind, die ausgewählt werden aus der Gruppe bestehend aus Folgendem: Inulinen, Fructooligosacchariden, resistenter Stärke, Lignin, Tannin, Zellulose, Hemizellulosen (einschließlich Xyloglucanen, Glucuronoxylanen, Arabinoglucuronoxylanen, Arabinoxylanen, Heteroxylanen, Mannanen und Beta-Glucanen), Psyllium, Polydextrose, Chitin, Chitosan, Pektinen (einschließlich Homogalacturonanen/Polygalacturonanen und Rhamnogalacturonanen I und II), Arabinan und Konnyaku/Konjak (Glucomannan).

12. Verfahren nach einem der vorhergehenden Ansprüche, wobei die anderen Enzyme, die für den Abbau der verschiedenen Ballaststoffe verantwortlich sind, aus der Gruppe ausgewählt werden, die bakterielle, archäale oder pilzliche Enzyme umfasst, die aus der Darmmikrobiota des Probanden stammen.

13. Computersoftwareprodukt, umfassend ein computerlesbares Medium, in dem Anweisungen gespeichert sind, wobei die Anweisungen, wenn von einem Datenprozessor gelesen, den Datenprozessor veranlassen, Daten zu empfangen, die aus einer Probe eines Probanden gesammelt wurden, und das Verfahren nach einem der Ansprüche 1-12 durchzuführen.

14. Vorrichtung zum Vorhersagen einer Reaktion eines Probanden auf verschiedene Ballaststoffe, wobei die Vorrichtung Folgendes umfasst: eine Benutzerschnittstelle, die zum Empfangen einer Probe eines Probanden ausgelegt ist; ein Modul zum Extrahieren spezifischer, aus der Mikrobiota stammender Nukleinsäuren aus der Probe des Probanden und zum Bestimmen und Quantifizieren der relativen Häufigkeit dieser spezifischen, aus der Mikrobiota stammenden Nukleinsäuren; und einen Datenprozessor mit einem computerlesbaren Medium, das das Computersoftwareprodukt nach Anspruch 13 speichert.

15. Vorrichtung nach Anspruch 14, ferner umfassend ein PCR-Kit zur Sequenzierung und Quantifizierung der relativen Häufigkeit der spezifischen, aus Mikrobiota stammenden Nukleinsäuren, die aus einer Trainingsdatenbank identifiziert wurden, wobei das PCR-Kit Folgendes umfasst:

a. Primer, die spezifisch für Nukleinsäuresequenzen sind, die aus der Trainingsdatenbank als diejenigen identifiziert wurden, die die Faserreaktion am besten vorhersagen; und
b. optional Primer, die spezifisch für Sequenzen aus den Genen und Stoffwechselwegen sind, die spezifisch für Schlüsselenzyme aus bakteriellen Acetat-, Propionat- und Butyrat-Produktionswegen sind, sowie für andere Enzyme, die für den Abbau der verschiedenen Ballaststoffe verantwortlich sind.

## Revendications

1. Méthode de prévision d'une réponse à différentes fibres alimentaires à partir de séquences d'acides nucléiques, ladite réponse à différentes fibres alimentaires étant définie sur la base des capacités de fermentation ou métaboliques du microbiote intestinal d'un sujet, évaluées par son taux de production d'acides gras à chaîne courte (AGCC) en réponse à ces différentes fibres alimentaires, la méthode étant appliquée aux données d'abondance d'acides nucléiques spécifiques dérivés du microbiote obtenus à partir d'un échantillon biologique stabilisé préalablement collecté dudit sujet, la méthode comprenant les étapes suivantes :

- isoler et extraire lesdits acides nucléiques spécifiques dérivés du microbiote à partir dudit échantillon biologique stabilisé, les séquences spécifiques desdits acides nucléiques spécifiques dérivés du microbiote étant identifiées à partir d'une base de données d'entraînement contenant des données de séquençage métagénomique du microbiote de sujets sains ou de sujets souffrant d'une maladie particulière, associées à des données mesurées *in vitro* concernant les taux de production de AGCC et d'autres métabolites de fermentation à partir de différentes fibres alimentaires chez ces sujets, lesdits AGCC et autres métabolites de fermentation étant choisis dans la liste comprenant le propionate, le butyrate, l'acétate, le lactate, le formate, le succinate, l'iso-butyrate, le valérate et l'iso-valérate ;
- déterminer et quantifier l'abondance relative desdits acides nucléiques spécifiques dérivés du microbiote à partir dudit échantillon biologique stabilisé afin de générer une première entrée, dans laquelle lesdits acides nucléiques spécifiques dérivés du microbiote consistent en des séquences de gènes spécifiques aux hydrolases de glycosides et/ou de polysaccharides capables d'hydrolyser ou de cliver des polysaccharides complexes de ces différentes fibres alimentaires, ainsi que des séquences génétiques spécifiques d'enzymes clés des voies bactériennes de production d'acétate, de propionate et de butyrate et/ou d'autres enzymes responsables de la dégradation de ces différentes fibres alimentaires ;
- appliquer un algorithme d'apprentissage automatique formé sur la base de données d'entraînement pour prédire, à partir de ces séquences d'acides nucléiques spécifiques dérivées du microbiote, le taux de production d'AGCC et d'autres métabolites de fermentation en réponse à une provocation par différentes fibres alimentaires, afin d'obtenir une réponse à ces différentes fibres alimentaires.

**2.** Méthode selon la revendication 1, **caractérisée en ce que** la base de données d'entraînement comprend des données classées selon un ensemble prédéterminé de groupes de classification, où l'évaluation des capacités de fermentation ou métaboliques du microbiote intestinal d'un sujet comprend la classification du sujet selon un type ou un phénotype spécifique.

**3.** La méthode selon l'une quelconque des revendications 1 à 2, **caractérisée en ce que** l'algorithme d'apprentissage automatique comprend en outre un ensemble d'algorithmes permettant de relier les données associées au microbiome du sujet à un certain état de santé, à un certain état physique ou à une réaction probable à une certaine thérapie.

**4.** La méthode selon l'une quelconque des revendications 1 à 3, comprenant en outre des étapes de stratification du sujet en fonction de différents plans de traitement, y compris des recommandations alimentaires personnalisées ou d'autres stratégies thérapeutiques visant à améliorer les capacités de fermentation du microbiote du sujet afin d'établir le profil du microbiome du sujet.

**5.** La méthode de la revendication 4, dans laquelle lesdites recommandations alimentaires personnalisées sont formulées en identifiant les apports en fibres alimentaires qui entraînent la production la plus élevée et la plus faible de métabolites d'intérêt par le microbiote du sujet, de manière à établir un produit de soins personnels pour le sujet.

**6.** La méthode de la revendication 4, dans laquelle lesdites stratégies thérapeutiques comprennent un ou plusieurs pré-biotiques, un ou plusieurs probiotiques, un ou plusieurs antibiotiques, ou tout autre médicament pour le traitement thérapeutique d'une maladie ou d'un trouble avec une implication métabolique et/ou immunologique et/ou inflammatoire.

**7.** La méthode de la revendication 4, dans laquelle les différents plans de traitement incluent une prédisposition à une maladie ou à un trouble avec une implication métabolique et/ou immunologique et/ou inflammatoire qui est choisie dans le groupe comprenant l'obésité, le syndrome ou les maladies métaboliques, le diabète sucré tel que le diabète de type 2, les troubles liés à la déficience en insuline, les troubles liés à la résistance à l'insuline, les troubles de la gluconéogenèse intestinale, les troubles de l'inflammation, la maladie inflammatoire de l'intestin, l'inflammation systémique ou locale dans le contexte de la neurodégénérescence, l'arthrite rhumatoïde, la dépression ou les troubles anxieux, l'intolérance alimentaire, la diarrhée, la constipation, la colite et l'entérite, les allergies et les applications de l'immunothérapie du cancer.

**8.** La méthode selon l'une quelconque des revendications précédentes, dans laquelle l'échantillon biologique du sujet est choisi dans le groupe comprenant : un écouvillon rectal, un échantillon fécal, une biopsie ou un échantillon de couche muqueuse.

**9.** La méthode selon l'une quelconque des revendications précédentes, dans laquelle la détermination et la quantification de l'abondance relative desdits acides nucléiques spécifiques dérivés du microbiote est effectuée soit par séquençage de l'ADN de gènes marqueurs spécifiques, y compris l'ARNr 16S, soit par séquençage de l'ADN de la métagénomique shotgun, soit par PCR ou qPCR de gènes spécifiques, soit par toute méthode de quantification des acides nucléiques, y compris l'électrophorèse capillaire.

**10.** La méthode selon l'une des revendications précédentes, dans laquelle ledit algorithme d'apprentissage automatique comprend une procédure d'apprentissage supervisé et éventuellement au moins une procédure choisie dans le groupe constitué par le regroupement, la machine à vecteurs de support, la modélisation linéaire, l'analyse des voisins les plus proches, l'apprentissage par arbre de décision, la procédure d'apprentissage d'ensemble, les réseaux neuronaux, le modèle probabiliste, le modèle graphique, le réseau bayésien et l'apprentissage par règles d'association.

**11.** La méthode selon l'une quelconque des revendications précédentes, dans laquelle les différentes fibres alimentaires sont des polysaccharides complexes ou d'autres ingrédients alimentaires choisis dans le groupe comprenant : inulines, fructo-oligosaccharide, amidon résistant, lignine, tanin, cellulose, hémicelluloses (y compris xyloglucanes, glucuronoxylanes, arabinoglucuronoxylanes, arabinoxylanes, hétéroxylanes, mannanes, et bêta-glucanes), psyllium, polydextrose, chitine, chitosane, les pectines (y compris les homogalacturonanes/polygalacturonanes et les rhamnogalacturonanes I et II), arabinane et konnyaku / konjac (glucomannane).

**12.** La méthode selon l'une quelconque des revendications précédentes, dans laquelle les autres enzymes responsables de la dégradation de ces différentes fibres alimentaires sont choisies dans le groupe comprenant des bactériennes, des archéennes ou des fongiques provenant du microbiote intestinal du sujet.

**13.** Produit logiciel informatique, comprenant un support lisible par ordinateur dans lequel sont stockées des instructions de programme, lesquelles instructions, lorsqu'elles sont lues par un processeur de données, amènent le processeur de données à recevoir des données collectées à partir de l'échantillon d'un sujet, et à exécuter la méthode selon l'une quelconque des revendications 1 à 12.

**14.** Appareil pour prédire la réponse d'un sujet à différentes fibres alimentaires, l'appareil comprenant : une interface utilisateur configurée pour recevoir un échantillon du sujet ; un module pour extraire des acides nucléiques spécifiques dérivés du microbiote de l'échantillon du sujet et pour déterminer et quantifier l'abondance relative de ces acides nucléiques spécifiques dérivés du microbiote ; et un processeur de données ayant un support lisible par ordinateur stockant le produit logiciel informatique de la revendication 13.

**15.** L'appareil selon la revendication 14, comprenant en outre un kit PCR pour le séquençage et la quantification de l'abondance relative desdits acides nucléiques spécifiques dérivés du microbiote identifiés à partir d'une base de données d'entraînement, ledit kit PCR comprenant

a. des amorces spécifiques pour les séquences d'acides nucléiques qui sont identifiées à partir de la base de données d'entraînement comme étant les plus prédictives de la réponse aux fibres; et
b. éventuellement, des amorces spécifiques des séquences des gènes et des voies métaboliques spécifiques aux enzymes clés des voies métaboliques bactériennes de production d'acétate, de propionate et de butyrate, ainsi que d'autres enzymes responsables de la dégradation desdites différentes fibres alimentaires.

Figure 1

Figure 2

Figure 3

Figure 4

Figure 5

Figure 6

6a

Figure 6

Figure 7

EP 4 035 175 B1

Figure 8

Figure 9

## Figure 10

Figure 11

EP 4 035 175 B1

Figure 12

Figure 13

Well pH over time

Figure 14

Figure 15

15a

$$x_p \qquad\qquad x_q \qquad\qquad x_r$$

$$\Big\downarrow \psi_{pi} \qquad\qquad \Big\downarrow \psi_{qj} \qquad\qquad \Big\downarrow \psi_{rk}$$

$$F \xrightarrow[\pi_{i1}]{x_i} O \xrightarrow[\pi_{j2}]{x_j} P \xrightarrow[\pi_{k3}]{x_k} SCFA$$

$$\Big\downarrow \gamma_M \qquad\qquad \Big\downarrow \gamma_P \qquad\qquad \Big\downarrow \gamma_{SCFA}$$

15b

$$\varnothing \qquad\qquad \varnothing \qquad\qquad \varnothing$$

$$F \xrightarrow[\Phi_{SCFA}(\mathbf{x})]{} SCFA$$

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2018106845 A1
- WO 2015166489 A2
- WO 2019046372 A1
- US 2018357375 A1, CUTCLIFFE COLLEEN

### Non-patent literature cited in the description

- **TINGTING CHEN et al.** Fiber-utilizing capacity varies in Prevotella-versus Bacteroides-dominated gut microbiota. *SCIENTIFIC REPORTS*, 01 June 2017, vol. 7 (1)
- **RILEY L HUGHES et al.** The Role of the Gut Microbiome in Predicting Response to Diet and the Development of Precision Nutrition Models. *ADVANCES IN NUTRITION*, 21 June 2019, vol. 10 (6), 953-978
- **BUGAUT, M.** Occurrence, absorption and metabolism of short chain fatty acids in the digestive tract of mammals. *Comp. Biochem. Physiol. Part B Comp. Biochem.*, 1987, vol. 86, 439-472
- **CHEN, T.** ; **LONG, W.** ; **ZHANG, C.** ; **LIU, S.** ; **ZHAO, L.** ; **HAMAKER, B.R.** Fiber-utilizing capacity varies in Prevotella - versus Bacteroides -dominated gut microbiota. *Sci. Rep.*, 2017, vol. 7, 2594
- **CHEN, W.L.K.** ; **EDINGTON, C.** ; **SUTER, E.** ; **YU, J.** ; **VELAZQUEZ, J.J.** ; **VELAZQUEZ, J.G.** ; **SHOCKLEY, M.** ; **LARGE, E.M.** ; **VENKATARAMANAN, R.** ; **HUGHES, D.J. et al.** Integrated gut/liver microphysiological systems elucidates inflammatory intertissue crosstalk. *Biotechnol. Bioeng.*, 2017, vol. 114, 2648-2659
- **COLE, J. R. et al.** The Ribosomal Database Project (RDP-II): sequences and tools for high-throughput rRNA analysis. *Nucleic Acids Res.*, 2005, vol. 33, D294-D296
- **DAVIE, J.R.** Inhibition of Histone Deacetylase Activity by Butyrate. *J. Nutr.*, 2003, vol. 133, 2485S-2493S
- **DE VADDER, F.** ; **KOVATCHEVA-DATCHARY, P.** ; **GONCALVES, D.** ; **VINERA, J.** ; **ZITOUN, C.** ; **DUCHAMPT, A.** ; **BÄCKHED, F.** ; **MITHIEUX, G.** Microbiota-Generated Metabolites Promote Metabolic Benefits via Gut-Brain Neural Circuits. *Cell*, 2014, vol. 156, 84-96
- **DUNCAN, S.H.** ; **BARCENILLA, A.** ; **STEWART, C.S.** ; **PRYDE, S.E.** ; **FLINT, H.J.** Acetate Utilization and Butyryl Coenzyme A (CoA):Acetate-CoA Transferase in Butyrate-Producing Bacteria from the Human Large Intestine. *Appl. Environ. Microbiol.*, 2002, vol. 68, 5186-5190
- **EDGAR, R. C.** UPARSE: highly accurate OTU sequences from microbial amplicon reads. *Nat. Methods*, 2013, vol. 10, 996-998
- **FILIPPIS, F.D.** ; **PASOLLI, E.** ; **TETT, A.** ; **TARALLO, S.** ; **NACCARATI, A.** ; **ANGELIS, M.D.** ; **NEVIANI, E.** ; **COCOLIN, L.** ; **GOBBETTI, M.** ; **SEGATA, N. et al.** Distinct Genetic and Functional Traits of Human Intestinal Prevotella copri Strains Are Associated with Different Habitual Diets. *Cell Host Microbe*, 2019, vol. 25, 444-453.e3
- **GURRY, T.** ; **GIBBONS, S.M.** ; **NGUYEN, L.T.T.** ; **KEARNEY, S.M.** ; **ANANTHAKRISHNAN, A.** ; **JIANG, X.** ; **DUVALLET, C.** ; **KASSAM, Z.** ; **ALM, E.J.** Predictability and persistence of prebiotic dietary supplementation in a healthy human cohort. *Sci. Rep.*, 2018, vol. 8, 12699
- **KUO, S.-M.** The Interplay Between Fiber and the Intestinal Microbiome in the Inflammatory Response. *Adv. Nutr. Int. Rev. J.*, 2013, vol. 4, 16-28
- **MACHIELS, K.** ; **JOOSSENS, M.** ; **SABINO, J.** ; **PRETER, V.D.** ; **ARIJS, I.** ; **EECKHAUT, V.** ; **BALLET, V.** ; **CLAES, K.** ; **IMMERSEEL, F.V.** ; **VERBEKE, K. et al.** A decrease of the butyrate-producing species Roseburia hominis and Faecalibacterium prausnitzii defines dysbiosis in patients with ulcerative colitis. *Gut*, 2013
- **MCNEIL, N.I.** ; **CUMMINGS, J.H.** ; **JAMES, W.P.** Short chain fatty acid absorption by the human large intestine. *Gut*, 1978, vol. 19, 819-822
- **PERRY, R.J.** ; **PENG, L.** ; **BARRY, N.A.** ; **CLINE, G.W.** ; **ZHANG, D.** ; **CARDONE, R.L.** ; **PETERSEN, K.F.** ; **KIBBEY, R.G.** ; **GOODMAN, A.L.** ; **SHULMAN, G.I.** Acetate mediates a microbiome-brain-$\beta$-cell axis to promote metabolic syndrome. *Nature*, 2016, vol. 534, 213-217
- **PREHEIM, S.P.** ; **PERROTTA, A.R.** ; **MARTIN-PLATERO, A.M.** ; **GUPTA, A.** ; **ALM, E.J.** Distribution-Based Clustering: Using Ecology to Refine the Operational Taxonomic Unit. *Appl Env. Microbiol*, 2013, vol. 79, 6593-6603
- **ROEDIGER, W.E.** Role of anaerobic bacteria in the metabolic welfare of the colonic mucosa in man. *Gut*, 1980, vol. 21, 793-798

- **SANNA, S.** ; **ZUYDAM, N.R. VAN** ; **MAHAJAN, A.** ; **KURILSHIKOV, A.** ; **VILA, A.V.** ; **VÕSA, U.** ; **MUJAGIC, Z.** ; **MASCLEE, A.A.M.** ; **JONKERS, D.M.A.E.** ; **OOSTING, M. et al.** Causal relationships among the gut microbiome, short-chain fatty acids and metabolic diseases. *Nat. Genet.*, 2019, vol. 1
- **SONNENBURG, E.D.** ; **ZHENG, H.** ; **JOGLEKAR, P.** ; **HIGGINBOTTOM, S.K.** ; **FIRBANK, S.J.** ; **BOLAM, D.N.** ; **SONNENBURG, J.L.** Specificity of Polysaccharide Use in Intestinal Bacteroides Species Determines Diet-Induced Microbiota Alterations. *Cell*, 2010, vol. 141, 1241-1252
- **ZHAO, L.** ; **ZHANG, F.** ; **DING, X.** ; **WU, G.** ; **LAM, Y.Y.** ; **WANG, X.** ; **FU, H.** ; **XUE, X.** ; **LU, C.** ; **MA, J. et al.** Gut bacteria selectively promoted by dietary fibers alleviate type 2 diabetes. *Science*, 2018, vol. 359, 1151-1156
- **ZIMMERMAN, M.A.** ; **SINGH, N.** ; **MARTIN, P.M.** ; **THANGARAJU, M.** ; **GANAPATHY, V.** ; **WALLER, J.L.** ; **SHI, H.** ; **ROBERTSON, K.D.** ; **MUNN, D.H.** ; **LIU, K.** Butyrate suppresses colonic inflammation through HDAC1-dependent Fas upregulation and Fas-mediated apoptosis of T cells. *Am. J. Physiol. - Gastrointest. Liver Physiol.*, 2012, vol. 302, G1405-G1415